# EUROPEAN PATENT SPECIFICATION

(11) **EP 3 922 232 B1**
(45) Date of publication and mention of the grant of the patent: **23.11.2022**
(21) Application number: 20752369.7
(22) Date of filing: 28.01.2020
(51) Int. Cl.: G16H 40/63

(54) **MEDICINE IDENTIFICATION SYSTEM, MEDICINE IDENTIFICATION DEVICE, MEDICINE IDENTIFICATION METHOD, AND PROGRAM**
SYSTEM ZUR IDENTIFIKATION VON ARZNEIMITTELN, VORRICHTUNG ZUR IDENTIFIKATION VON ARZNEIMITTELN, VERFAHREN ZUR IDENTIFIKATION VON ARZNEIMITTELN UND PROGRAMM
SYSTÈME D'IDENTIFICATION DE MÉDICAMENT, DISPOSITIF D'IDENTIFICATION DE MÉDICAMENT, PROCÉDÉ D'IDENTIFICATION DE MÉDICAMENT, ET PROGRAMME

(30) Priority: 08.02.2019 JP 2019021802
(43) Date of publication of application: 15.12.2021
(73) Proprietor: FUJIFILM Toyama Chemical Co., Ltd., Chuo-ku Tokyo 104-0031 (JP)
(72) Inventor: IWAMI, Kazuchika, Tokyo 104-0031 (JP)
(74) Representative: DehnsGermany Partnerschaft von Patentanwälten
(86) International application number: PCT/JP2020/002946
(87) International publication number: WO 2020/162262

(56) References cited:
- JP-A- 2015 535 108
- JP-A- 2017 194 413
- US-A1- 2014 119 644

## Description

### BACKGROUND OF THE INVENTION

### 1. Field of the Invention

The present invention relates to a medicine identification system, a medicine identification device, a medicine identification method, and a program, and in particular to a technology of identifying a medicine to be identified by means of a simple device.

### 2. Description of the Related Art

Conventionally, a system has been proposed that acquires an image for identifying a tablet (medicine) by using a mobile device such as a smartphone including a camera, and identifies the medicine based on the image thus acquired (Patent Literature 1).

The surface (surface of a tray on which the medicine is to be placed) disclosed in Patent Literature 1 includes a rectangular background region in which the medicine is to be placed, and a boundary region surrounding the background region. The background region has a high-density colored checker board pattern to permit distinction between the background and the medicine, while the boundary region has a color property of which color is known (white). Meanwhile, in the boundary region (four regions corresponding to four corners of the rectangular background region), four known targets (yellow circle) are provided, with an anchor point being provided at the center of each of the targets.

The four targets provided in the boundary region have a function of assisting a user in imaging the medicine with the mobile device, by allowing the mobile device to be in the preset position and attitude.

On a screen of the mobile device, target fields corresponding to the four targets are displayed in advance. Thus, imaging of the medicine while standardizing the size and shape thereof is enabled through determining the position and attitude of the mobile device such that the four targets with the anchor points are properly positioned in the four target fields displayed.

In addition, the system disclosed in Patent Literature 1 corrects the color of a pixel belonging to the medicine to produce the color of the pixel captured under an ideal lighting condition, based on the known color of the boundary region.

### Citation List

Patent Literature 1: Japanese Translation of PCT International Application Publication No. 2015-535108. Further relevant documents: Document US 2014/119644 A1 discloses a mobile medicine identification device and document JP 2017 194413 A discloses a drug recognition system that features illumination units that can illuminate a medication in question from a plurality of illumination directions. However, none of the documents discloses the generation of a plurality of illumination directions for image capture by changing of the light-emitting region on the display of a mobile terminal device.

### SUMMARY OF THE INVENTION

Patent Literature 1 discloses identifying a medicine based on the shape, size, color, and imprint (embossed or printed character(s) and numeral(s)), but does not provide any disclosure regarding an emphasis process for emphasizing an embossed mark or a printed mark.

Patent Literature 1 also discloses a system for assisting adjustment of the position and attitude of a camera-equipped mobile device so as to enable imaging of the medicine with the mobile device while standardizing the shape and size thereof; however, there is a problem of requiring a user to adjust the position and attitude of the mobile device before imaging, resulting in cumbersome imaging.

The present invention has been made in view of the aforementioned circumstances, and an objective of the present invention is to provide a medicine identification system, a medicine identification device, a medicine identification method, and a program that enable favorable identification of a medicine by means of a simple device employing a camera-equipped mobile terminal.

In order to achieve the aforementioned objective, in the invention according to one aspect, a medicine identification system includes: a tray configured to have an indicator used for standardizing size and shape of a medicine, on which a medicine to be identified is to be placed; a camera-equipped mobile terminal configured to include a camera on the same face as a display; and a medicine identification device configured to identify the medicine based on an image taken by the camera-equipped mobile terminal, in which the camera-equipped mobile terminal is provided with: a display control unit configured to, upon reception of an instruction input for medicine identification, change a light-emitting region on the display and change for a plurality of times an illuminating direction with respect to the medicine placed on the tray; and an image acquiring unit configured to cause the camera to image for a plurality of times the medicine with different illuminating directions through changing of the light-emitting region on the display, to thereby acquire a plurality of first images of the medicine, and the medicine identification device is provided with: a standardizing unit configured to standardize each of the plurality of first images based on the indicator captured in the plurality of first images; an image extracting unit configured to extract a medicine region image from each of the plurality of first images thus standardized; an image processing unit configured to generate, based on a plurality of medicine region images thus extracted, a second image having undergone an emphasis process for emphasizing an embossed mark or a printed mark provided on the medicine; and a medicine identifying unit configured to identify the medicine based on the second image thus generated.

According to the one aspect of the present invention, the camera-equipped mobile terminal images the medicine placed on the tray together with the indicator provided on the tray. During imaging of the medicine, the display on the same face as the camera is used as illuminating means. In particular, a plurality of images (first images) are acquired by causing the camera to image for a plurality of times the medicine with different illuminating directions, in such a way that an illuminating direction with respect to the medicine placed on the tray is changed for a plurality of times through changing of the light-emitting region on the display. The standardizing unit standardizes each of the plurality of first images based on the indicator captured in the plurality of first images, thereby making the first images independent of the position and attitude of the camera during imaging. The image extracting unit extracts a medicine region image from each of the plurality of first images thus standardized, and the image processing unit generates, based on a plurality of medicine region images thus extracted, an image (second image) having undergone an emphasis process for emphasizing an embossed mark or a printed mark provided on the medicine. The second image shows the size and shape of the medicine, and also has an embossed mark or a printed mark provided on the medicine being emphasized, thereby enabling favorable identification of the medicine. Furthermore, the one aspect of the present invention eliminates the need for dedicated lighting device, camera, and the like, and thus enables identification of a medicine by means of a simple device.

In the medicine identification system according to another aspect of the present invention, it is preferable that the image processing unit acquires a plurality of edge images from the plurality of medicine region images thus extracted by using edge extraction filters of respective directions corresponding to the illuminating directions, and generates the second image by using the plurality of edge images.

In the medicine identification system according to still another aspect of the present invention: the tray has a grid-shaped partition of which size and shape are known; the medicine is placed on a placement face partitioned by the grid-shaped partition; and the grid-shaped partition serves also as the indicator. By placing a plurality of medicines of different types on the plurality of placement faces partitioned by the grid-shaped partition, imaging for identification of the plurality of medicines can be carried out at once, and the medicine region images can be easily extracted (trimmed) by using the grid-shaped partition as a guide.

In the medicine identification system according to still another aspect of the present invention, it is preferable that color of the placement face of the tray for the medicine partitioned by the grid-shaped partition is different in at least one of hue, brightness, and saturation, for every placement face or for every plural placement faces. It is preferable to place the medicine by choosing the placement face of which color is different from the color of the medicine in one of hue, brightness, and saturation. This is for facilitating distinction between the medicine region images and the placement face (background), and in turn making it easy to trim the medicine region images by the image extracting unit.

In the medicine identification system according to still another aspect of the present invention, it is preferable that: the image acquiring unit acquires, as the plurality of first images, three or more images with different illuminating directions and an image in a state in which the display is turned off; and the medicine identification device is provided with a generating unit configured to subtract the image in a state in which the display is turned off from each of the three or more images, to generate the plurality of first images with elimination of influence of ambient light. As a result, the medicine region images can be reproduced with the original color of the medicine without the influence of ambient light.

In the medicine identification system according to still another aspect of the present invention: it is preferable that the display includes a polarizing plate; and the camera includes a polarizing plate installed in front of an imaging lens, a polarization direction of the polarizing plate being different by 90° from a polarization direction of light emitted from the display. This enables an image to be taken with elimination of a specular reflection component.

In the medicine identification system according to still another aspect of the present invention, it is preferable that the medicine identifying unit includes: a feature quantity calculating unit configured to calculate a feature quantity of the second image; and an inferring unit configured to infer which one of registered medicines resembles the medicine based on the feature quantity of the second image.

In the medicine identification system according to still another aspect of the present invention, it is preferable that the feature quantity calculating unit and the inferring unit include a learned convolutional neural network obtained through learning of each of the registered medicines by using as teacher data the second images corresponding to the registered medicines and medicine identification information for identifying the registered medicines.

In the medicine identification system according to still another aspect of the present invention, it is preferable that the medicine identifying unit identifies the medicine through template matching between images of the registered medicines narrowed down by inference by the inferring unit and the second image.

In the medicine identification system according to still another aspect of the present invention, it is preferable that the tray serves also as a protection cover for protecting the camera-equipped mobile terminal.

In the invention according to further aspect, a medicine identification device for identifying a medicine to be identified based on an image taken by a camera-equipped mobile terminal, in which the camera-equipped mobile terminal is configured to: include a camera on the same face as a display; when imaging the medicine to be identified placed on a tray having an indicator used for standardizing size and shape of a medicine, change a light-emitting region on the display; change for a plurality of times an illuminating direction with respect to the medicine placed on the tray; and image by means of the camera for a plurality of times the medicine with different illuminating directions through changing of the light-emitting region on the display, to acquire a plurality of first images of the medicine, the medicine identification device includes: an image accepting unit configured to accept the plurality of first images of the medicine from the camera-equipped mobile terminal; a standardizing unit configured to standardize each of the plurality of first images based on the indicator captured in the plurality of first images; an image extracting unit configured to extract a medicine region image from each of the plurality of first images thus standardized; an image processing unit configured to generate, based on a plurality of medicine region images thus extracted, a second image having undergone an emphasis process for emphasizing an embossed mark or a printed mark provided on the medicine; and a medicine identifying unit configured to identify the medicine based on the second image thus generated.

In the invention according to still further aspect, a medicine identification method for identifying a medicine to be identified placed on a tray having an indicator used for standardizing size and shape of a medicine includes: a first step in which a display control unit changes a light-emitting region on a display of a camera-equipped mobile terminal including a camera on the same face as the display, to thereby change for a plurality of times an illuminating direction with respect to the medicine placed on the tray; a second step in which an image acquiring unit causes the camera to image for a plurality of times the medicine with different illuminating directions through changing of the light-emitting region on the display, to thereby acquire a plurality of first images of the medicine; a third step in which a standardizing unit standardizes each of the plurality of first images based on the indicator captured in the plurality of first images; a fourth step in which an image extracting unit extracts a medicine region image from each of the plurality of first images thus standardized; a fifth step in which an image extracting unit generates, based on a plurality of medicine region images thus extracted, a second image having undergone an emphasis process for emphasizing an embossed mark or a printed mark provided on the medicine; and a sixth step in which a medicine identifying unit identifies the medicine based on the second image thus generated.

In the medicine identification method according to still further aspect of the present invention, it is preferable that the fifth step acquires a plurality of edge images from the plurality of medicine region images thus extracted by using edge extraction filters of respective directions corresponding to the illuminating directions, and generates the second image by using the plurality of edge images.

In the medicine identification method according to still further aspect of the present invention: it is preferable that the tray has a grid-shaped partition of which size and shape are known; the medicine is placed on a placement face partitioned by the grid-shaped partition; and the grid-shaped partition serves also as the indicator.

In the medicine identification method according to still further aspect of the present invention, it is preferable that color of the placement face of the tray for the medicine partitioned by the grid-shaped partition is different in at least one of hue, brightness, and saturation, for every placement face or for every plural placement faces.

In the medicine identification method according to still further aspect of the present invention, it is preferable that the second step acquires, as the plurality of first images, three or more images with different illuminating directions and an image in a state in which the display is turned off, and includes a step in which a generating unit subtracts the image in a state in which the display is turned off from each of the three or more images, to thereby generate the plurality of first images with elimination of influence of ambient light.

In the medicine identification method according to still further aspect of the present invention, it is preferable that, after the medicine placed on the tray has been turned over, the first step to the fifth step carry out identical procedures respectively with respect to the medicine thus turned over; and the fifth step identifies the medicine based on the second images generated respectively for the medicine before turning over and the medicine after turning over.

In the invention according to yet further aspect, a program installed on the camera-equipped mobile terminal constituting the aforementioned medicine identification system causes the camera-equipped mobile terminal to execute: a display control function configured to, upon reception of an instruction input for medicine identification, change a light-emitting region on the display and change for a plurality of times an illuminating direction with respect to the medicine placed on the tray; and an image acquiring function configured to cause the camera to image for a plurality of times the medicine with different illuminating directions through changing of the light-emitting region on the display, to thereby acquire a plurality of first images of the medicine.

It is preferable that the program according to yet further aspect of the present invention causes the camera-equipped mobile terminal to execute: a standardizing function configured to standardize each of the plurality of first images based on the indicator captured in the plurality of first images; an image extracting function configured to extract a medicine region image from each of the plurality of first images thus standardized; an image processing function configured to generate, based on a plurality of medicine region images thus extracted, a second image having undergone an emphasis process for emphasizing an embossed mark or a printed mark provided on the medicine; and a medicine identifying function configured to identify the medicine based on the second image thus generated.

According to the present invention, by imaging the medicine placed on the tray together with the indicator provided on the tray by means of the camera-equipped mobile terminal, the image (size and shape of the medicine) can be standardized based on the indicator captured in the image. In addition, by using the display on the same face as the camera as illuminating means, and in particular changing the light-emitting region on the display, imaging of the medicine placed on the tray with different illuminating directions with the camera is enabled. And then the image (second image) having undergone the emphasis process for an embossed mark or a printed mark provided on the medicine is generated based on the plurality of images (first images) taken with different illuminating directions, the second image enabling favorable identification of the medicine.

### BRIEF DESCRIPTION OF THE DRAWINGS

Fig. 1 is a system configuration diagram showing an embodiment of a medicine identification system according to the present invention.
Fig. 2 is an external view of a smartphone constituting the medicine identification system shown in Fig. 1
Fig. 3 is a block diagram showing an internal configuration of the smartphone shown in Fig. 2
Fig. 4 is a main-section block diagram showing an electrical configuration of the medicine identification system shown in Fig. 1.
Fig. 5 is a diagram showing a configuration of a tray constituting the medicine identification system shown in Fig. 1, and a state of imaging with the smartphone a medicine placed on the tray.
Fig. 6 is a diagram showing a light-emitting region on a display to be controlled in the case of imaging the medicine placed on the tray for five times.
Fig. 7 is a block diagram showing a standardizing unit, an image extracting unit, and an image processing unit in the server shown in Fig. 4.
Fig. 8 is a diagram showing an example of five first images after standardization.
Fig. 9 is a schematic view of a cross-sectional structure of the medicine taken along an x-y plane passing through the center of the medicine.
Fig. 10 is a diagram showing an example of a Sobel filter used for edge extraction by an edge image generating unit.
Fig. 11 is a diagram showing an outline of a medicine identification process by a medicine identifying unit according to a first embodiment of the medicine identifying unit shown in Fig. 4.
Fig. 12 is a functional block diagram showing main functions of the medicine identifying unit according to the first embodiment.
Fig. 13 is a block diagram showing a medicine identifying unit according to a second embodiment.
Fig. 14 is a diagram showing another embodiment of the tray used in the medicine identification system according to the present invention.
Fig. 15 is a flowchart showing an embodiment of a medicine identification method according to the present invention.

### DESCRIPTION OF THE PREFERRED EMBODIMENTS

Hereinafter, preferred embodiments of a medicine identification system, a medicine identification device, a medicine identification method, and the program according to the present invention are described with reference to the attached drawings.

### Configuration of Medicine Identification System

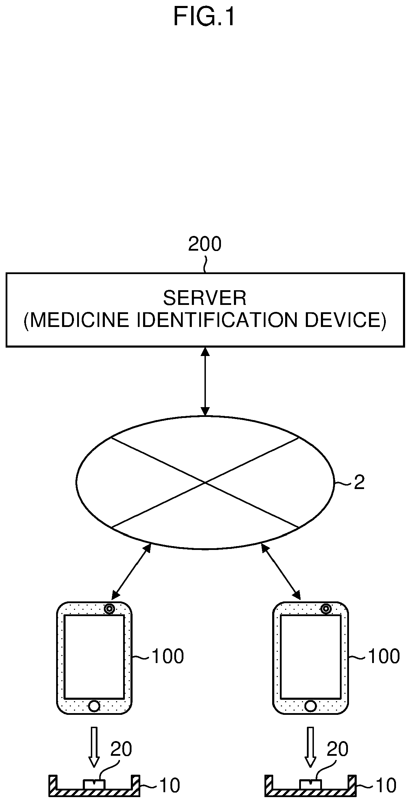

Figure 1 is a system configuration diagram showing an embodiment of a medicine identification system according to the present invention.

As shown in Figure 1, the medicine identification system is configured with a tray 10, a smartphone 100 which is a camera-equipped mobile terminal, and a server 200 functioning as the medicine identification device, and the smartphone 100 and the server 200 are connected to each other in such a way that data communication is possible, via a network 2 such as internet, LAN (Local Area Network), and the like.

The tray 10, on which one or more medicines 20 to be identified is to be placed, has an indicator used for standardizing size and shape of a medicine as described later. In addition, it is preferable that the tray 10 serves also as a protection cover for protecting the smartphone 100.

The smartphone 100 includes a camera (front camera) on the same face as the display, and images the medicine 20 on the tray 10 by means of the camera. The smartphone 100 transmits an image of the medicine 20 thus taken to the server 200 via the network 2.

The server 200 identifies the medicine 20 based on the image of the medicine 20 uploaded from the smartphone 100, and transmits an identification result (for example, medicine identification information composed of a drug name, a trade name, an abbreviation, or a combination thereof) to the smartphone 100 having transmitted the image of the medicine 20.

Incidentally, identification code information for identifying classification of the medicine is provided on the surface of the medicine (tablet). The identification code information is provided typically by means of embossing or typing (printing).

The server 200 has an improved medicine identification capacity through use of the identification code information provided on the medicine, as described later in detail.

Note that an embossed mark provided on the medicine refers to the identification code information provided through formation of a groove, which is a concave region, on the surface of the medicine. The groove is not limited to that formed by engraving the surface, and may also be formed by pressing the surface. In addition, the embossed mark may also include those with no identification function, such as a score line.

Note also that a printed mark provided on the medicine refers to the identification code information provided through application of edible ink, etc. in a contact or noncontact manner on the surface of the medicine. As used herein, the expression "provided by typing" is synonymous with "provided by printing".

### <Configuration of Smartphone>

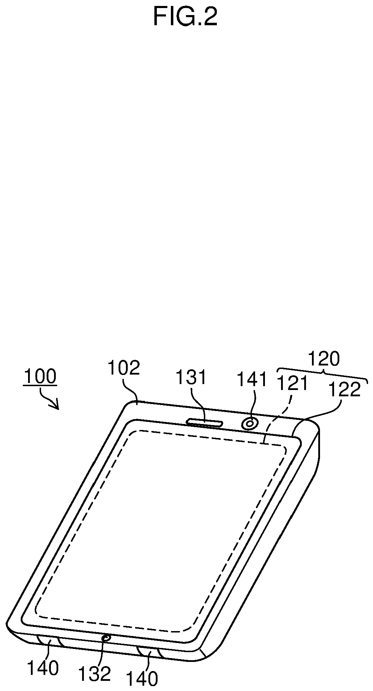

The smartphone 100 shown in Figure 2 has a flat plate-like housing 102, and a display 120, which is composed of a display panel 121 as a display unit and an operation panel 122 as an input unit in an integral manner, is provided on one side of the housing 102. The display panel 121 is configured with a liquid crystal panel, and thus the display 120 of the present example is a liquid crystal display.

In addition, the housing 102 includes a speaker 131, a microphone 132, an operation unit 140, and a camera unit 141. The camera unit 141 is a camera (front camera) provided on the same face as the display 120. The smartphone 100 is provided also with a camera (rear camera) not illustrated, not on a front face of the housing 102 on which the display 120 is provided, but on a rear face of the housing 102. Note that another lens (adapter lens for short-distance imaging) may be attached in front of a lens of the camera unit 141.

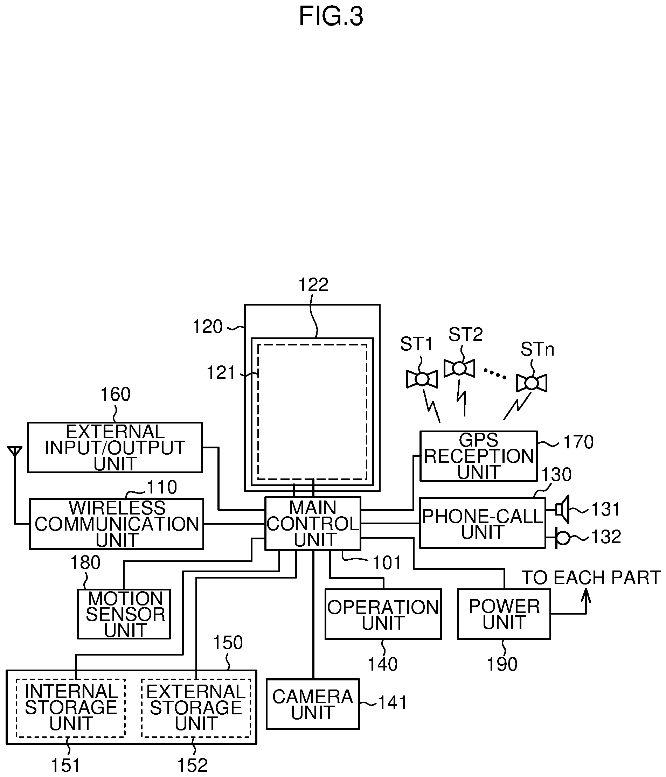

Figure 3 is a block diagram showing an internal configuration of the smartphone 100 shown in Figure 2.

As shown in Figure 3, the smartphone 100 includes, as main constituent elements, a wireless communication unit 110, the display 120, a phone-call unit 130, the operation unit 140, the camera unit 141, a storage unit 150, an external input/output unit 160 (output unit), a GPS (Global Positioning System) reception unit 170, a motion sensor unit 180, a power unit 190, and a main control unit 101. In addition, the smartphone 100 has, as a main function, a wireless communication function for carrying out mobile wireless communication via a base station device and a mobile communication network.

The wireless communication unit 110 carries out wireless communication with the base station device connected to the mobile communication network, according to an instruction from the main control unit 101. By means of the wireless communication, transmission/reception of various types of file data such as sound data and image data, electronic mail data, and the like; and reception of web data, streaming data and the like are carried out.

The display 120 is a so-called touch-screen-equipped display including the operation panel 122 provided on a screen of the display panel 121 that, under control of the main control unit 101, visually transmits information to a user by displaying images (still image and video), character information, and the like, and detects a user operation in response to the information thus displayed.

The display panel 121 uses an LCD (Liquid Crystal Display) as a display device. Note that the display panel 121 is not limited to the LCD and may also be, for example, an OLED (Organic Light Emitting Diode).

The operation panel 122 is a device that is provided in a state such that an image displayed on a display face of the display panel 121 is visually recognizable, and detects one or more coordinates operated by a user's finger or a stylus. When the device is operated by the user's finger or the stylus, the operation panel 122 outputs to the main control unit 101 a detection signal generated due to the operation. Then, the main control unit 101 detects an operated position (coordinate) on the display panel 121 based on the detection signal thus received.

The phone-call unit 130 including the speaker 131 and the microphone 132 converts user's sound being input through the microphone 132 into sound data processable by the main control unit 101 and then outputs to the main control unit 101, and decodes sound data received by the wireless communication unit 110 or the external input/output unit 160 and then outputs from the speaker 131.

The operation unit 140 is a hardware key employing a key switch or the like, that receives an instruction from the user. For example, as shown in Figure 2, the operation unit 140 is a push-button type switch installed on a lateral face of the housing 102 of the smartphone 100, that reaches an active state when pressed by a finger or the like, and reaches an inactive state due to a restorative force of a spring or the like when the finger is released.

The storage unit 150 stores a control program and control data of the main control unit 101, address data containing names, telephone numbers, etc. of communication counterparts in association with each other, data of transmitted and received electronic mails, web data downloaded through web browsing, downloaded content data, and the like, and also temporarily stores streaming data and the like.

In addition, the storage unit 150 includes an internal storage unit 151 and an external storage unit 152 having a detachable external memory slot. Note that the internal storage unit 151 and the external storage unit 152 constituting the storage unit 150 are each realized by means of a storage medium such as flash memory type memory, hard disk type memory, multimedia card micro type memory, card type memory, RAM (Random Access Memory), ROM (Read Only Memory), or the like.

The external input/output unit 160 serves as an interface for all external devices to be connected to the smartphone 100, and is connected directly or indirectly to other external devices via communication or the like (for example, USB (Universal Serial Bus), IEEE1394, etc.) or a network (for example, wireless LAN (Local Area Network), Bluetooth (Registered Trademark).

The GPS reception unit 170, according to an instruction from the main control unit 101, receives GPS signals transmitted from GPS satellites ST1, ST2, ... STn, executes a positioning computing process based on the plurality of GPS signals thus received, and obtains position information (GPS information) identified based on latitude, longitude, and altitude of the smartphone 100. In a case in which position information can be obtained from the wireless communication unit 110 and/or the external input/output unit 160 (for example, wireless LAN), the GPS reception unit 170 may also detect a position by using the position information.

The motion sensor unit 180 is provided with, for example, a triaxial acceleration sensor and the like, and detects physical movement of the smartphone 100 according to an instruction from the main control unit 101. Moving direction and acceleration of the smartphone 100 are detected through detection of the physical movement of the smartphone 100. A result of the detection is output to the main control unit 101.

The power unit 190 supplies electricity stored in a battery (not illustrated) to each part of the smartphone 100, according to an instruction from the main control unit 101.

The main control unit 101 is provided with a microprocessor, and operates according to the control program and the control data stored in the storage unit 150, to integrally control each part of the smartphone 100. In addition, the main control unit 101 is provided with a mobile communication control function for controlling each part related to communication and a software processing function, in order to carry out voice communication and data communication through the wireless communication unit 110.

The software processing function is realized by the main control unit 101 operating according to software stored in the storage unit 150. The software processing function is exemplified by an electronic mail function for transmitting and receiving electronic mails through control of the external input/output unit 160, a web browsing function for browsing web pages, a function for using the medicine identification system according to the present invention, and the like. The function for using the medicine identification system according to the present invention can be realized by downloading relevant software (the program according to the present invention) from the server 200 functioning as the medicine identification device, a website of a provider that runs the server 200, or the like.

Furthermore, the main control unit 101 is provided with an image processing function of, for example, displaying a video on the display 120 based on image data (data of still image and video) such as received data, downloaded streaming data, and the like.

Moreover, the main control unit 101 executes a display control with respect to the display 120, and an operation detection control for detecting a user operation through the operation unit 140 and the operation panel 122.

The camera unit 141 is capable of, under control of the main control unit 101: converting data acquired by imaging to, for example, compressed image data such as JPEG (Joint Photographic Experts Group); storing the image data to the storage unit 150; and outputting the image data through the external input/output unit 160 or the wireless communication unit 110.

In addition, the camera unit 141 may be used for various functions of the smartphone 100. In the present example, in the case of identification of a medicine, the camera unit 141 is used for imaging the medicine. It is also possible to use an image from the camera unit 141 in software.

### <Electrical Configuration of Medicine Identification System>

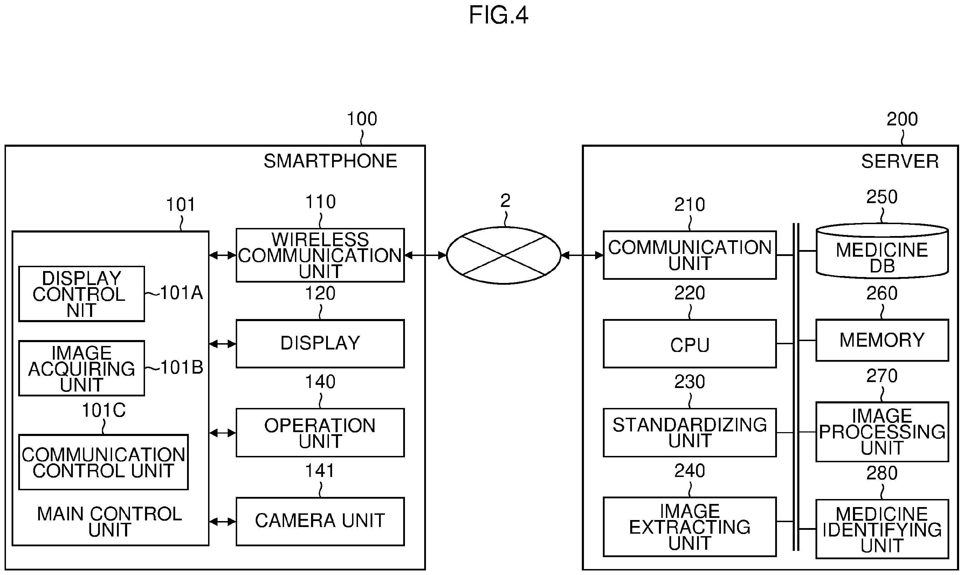

Figure 4 is a main-section block diagram showing an electrical configuration of the medicine identification system.

The main control unit 101 of the smartphone 100 has the program (application) according to the present invention installed, and functions as a display control unit 101A, an image acquiring unit 101B, and a communication control unit 101C through execution of the application.

The display control unit 101A controls the display 120 as illuminating means during imaging of a medicine, in particular by carrying out a control of changing for a plurality of times a light-emitting region on the display 120 to change an illuminating direction with respect to the medicine 20 placed on the tray 10.

The image acquiring unit 101B causes the camera unit 141 to image for a plurality of times the medicine 20 with different illuminating directions through changing of the light-emitting region on the display 120, to thereby acquire from the camera unit 141 a plurality of images (first images) of the medicine.

The communication control unit 101C transmits the plurality of first images thus acquired by the image acquiring unit 101B to the server 200 via the wireless communication unit 110 and the network 2, and acquires an identification result of the medicine 20 identified by the server 200 based on the plurality of first images via the network 2 and the wireless communication unit 110.

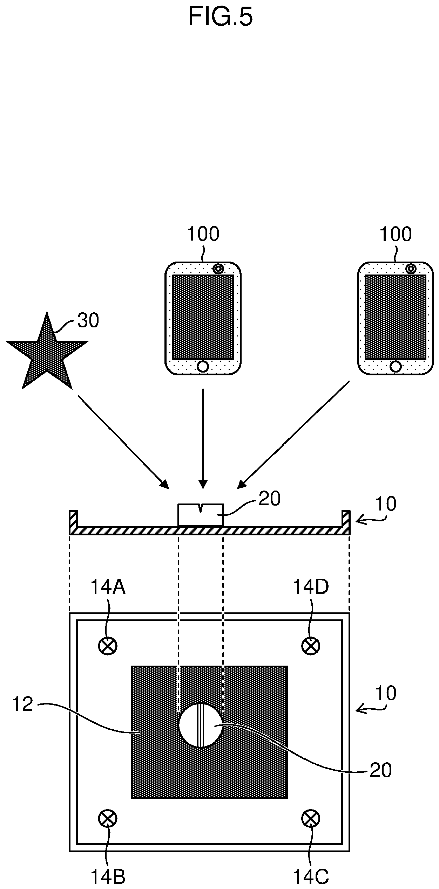

Figure 5 is a diagram showing a configuration of the tray 10, and a state of imaging with the smartphone 100 the medicine 20 placed on the tray 10.

As shown in Fig. 5, a placement face (in the present example, a rectangular placement face to be a background for the medicine) 12 on which the medicine 20 is placed, and indicators (four indicators) 14A, 14B, 14C, 14D used for standardizing the size and shape of the medicine, are provided on the tray 10.

It is preferable that the placement face 12 has a specific background color (color different from the color of the medicine) or a specific fine background pattern, in order to facilitate extraction (trimming) of an image of a region corresponding to the medicine (medicine region) from the image taken. It is preferable that the background color for the medicine is different from the color of the medicine in at least one of hue, brightness, and saturation.

The four indicators 14A, 14B, 14C, and 14D have a known positional relationship with each other. Note that, instead of the four indicators 14A, 14B, 14C, and 14D, for example, information relating to the shape of the placement face 12 (coordinates of four corners, lengths of four sides, etc. of the rectangular placement face 12) may be used as the indicator.

In the case of imaging the medicine 20 placed on the tray 10 with the smartphone 100, the imaging needs to be carried out with the position and attitude of the smartphone 100 (camera unit 141) being selected such that the four indicators 14A, 14B, 14C, and 14D are captured within an imaging range of the camera unit 141. Note that, although it is preferable that the imaging is carried out with the position and attitude with which the medicine 20 is right in front of the camera unit 141 and captured in large size, the position and attitude of the camera unit 141 are only required to be selected such that the four indicators 14A, 14B, 14C, and 14D are captured within an imaging range.

In addition, in the case of imaging the medicine 20 placed on the tray 10 with the smartphone 100, the tray 10 with the medicine 20 placed thereon is imaged by the camera unit 141 for a plurality of times (five times in the present example) while using the display 120 on the same face as the camera unit 141 as illuminating means.

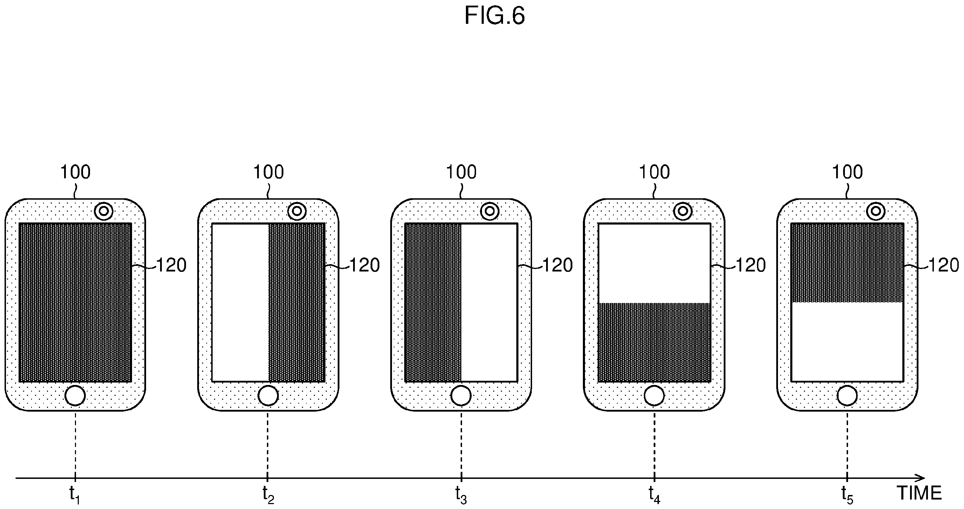

Figure 6 is a diagram showing a light-emitting region on the display 120 to be controlled in the case of imaging the medicine 20 placed on the tray 10 for five times.

For example when the application according to the present example is activated and then receives an imaging instruction input (instruction input for medicine identification) from the operation unit, the display control unit 101A changes the light-emitting region on the display 120 coincidentally with five imaging timings at time t₁, time t₂, time t₃, time t₄, and time ts as shown in Figure 6.

At the imaging timing at time t₁, the medicine 20 is imaged in a state in which the display 120 is turned off and ambient light 30 (Figure 5) is the only illuminating light.

Note that, since the display 120 of the present example is a liquid crystal display employing a liquid crystal panel as the display panel 121, "the display 120 is turned off" means that the transmitted light amount of the entire liquid crystal panel is minimized (black color is displayed).

Subsequently, at the imaging timing at time t₂, the medicine 20 is imaged in a state in which the light-emitting region on the display 120 is a left half of the display 120, and the ambient light 30 and illuminating light from the display 120 are mixed. As used herein, "the light-emitting region on the display 120 is a left half of the display 120" means that the transmitted light amount of a left half of the liquid crystal panel is increased (preferably maximized) while the transmitted light amount of a right half is minimized.

In a similar manner, the medicine 20 is imaged at respective imaging timings: in a state in which the light-emitting region on the display 120 is a right half of the display 120 at the imaging timing at time t₃; in a state in which the light-emitting region on the display 120 is an upper half of the display 120 at the imaging timing at time t₄; and in a state in which the light-emitting region on the display 120 is a lower half of the display 120 at the imaging timing at time t₅.

The image acquiring unit 101B operates the camera unit 141 to image at the five imaging timings at time t₁, time t₂, time t₃, time t₄, and time t₅, and acquires five first images taken of the medicine 20 from the camera unit 141. Since the light-emitting region on the display 120 is the left half, the right half, the upper half, and the lower half of the display 120 at time t₂, time t₃, time t₄, and time ts respectively, the illuminating light from the display 120 to the medicine 20 is thus different in the illuminating direction. In addition, the first images acquired by imaging the medicine 20 illuminated with the illuminating light of respective different illuminating directions each have luminance unevenness generated along the illuminating direction, resulting in, when the medicine 20 is provided with an embossed mark, difference in the way in which a shadow of the embossed mark appears.

It is preferable that a time interval between the imaging timings is short, so that a change in the position and attitude of the camera unit 141 may be minimized during five sessions of imaging.

Since the display 120 is a liquid crystal display with a polarizing plate, the illuminating light emitted from the display 120 is a polarized light corresponding to the polarizing plate. Given this, it is preferable to install a polarizing plate (polarizing filter) in front of an imaging lens of the camera unit 141, a polarization direction of the polarizing plate being different by 90° from a polarization direction of light emitted from the display 120.

By installing the polarizing filter at the camera unit 141, the camera unit 141 is enabled to take the first images with elimination of a specular reflection component.

Note that when the medicine 20 is provided with an embossed mark or a printed mark, it is preferable that the medicine 20 is placed on the tray 10 in such a way that a face with the embossed mark or the printed mark is directed upward. In the case in which the medicine 20 is provided with an embossed mark or a printed mark on both faces, it is preferable that the medicine 20 is placed on the tray 10 in such a way that a side with the embossed mark or the printed mark functioning better as the identification code information is directed upward.

### <Server 200>

The server 200 shown in Figure 4 functions as the medicine identification device, and is configured primarily with a communication unit 210, a CPU (Central Processing Unit) 220, a standardizing unit 230, an image extracting unit 240, a medicine DB (database) 250, a memory 260, an image processing unit 270, and a medicine identifying unit 280.

The CPU 220 integrally controls each part of the server 200, and, together with the communication unit 210 functioning as the image accepting unit that accepts the plurality of first images transmitted from the smartphone 100, causes the standardizing unit 230, the image extracting unit 240, the image processing unit 270, and the medicine identifying unit 280 to execute each process on the plurality of first images thus accepted. The CPU 220 then transmits an identification result of the medicine to the smartphone 100 having transmitted the first images, via the communication unit 210.

The medicine DB 250 is a component that registers and manages images of medicines (images of obverse sides and reverse sides of medicines) in association with the medicine identification information such as medicine names. The medicine images of medicines (registered medicines) registered in the medicine DB 250 are used as teacher data in a case of teaching a machine learning device that checks which one of registered medicines resembles a medicine to be identified, or as template images in a case of identifying a medicine to be identified through template matching with an image of the medicine to be identified.

The memory 260 includes a storage unit in which a program providing a medicine identification service is stored, and a portion serving as a workspace for the CPU 220.

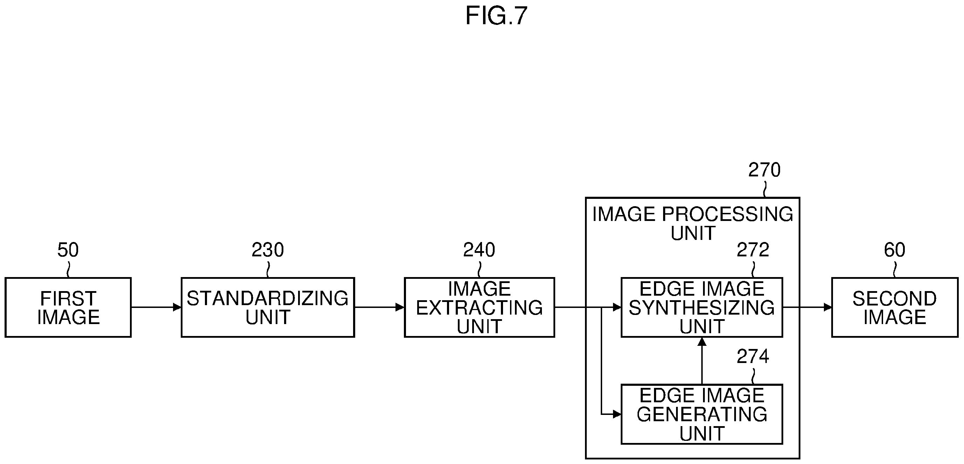

Figure 7 is a block diagram showing the standardizing unit 230, the image extracting unit 240, and the image processing unit 270 in the server 200 shown in Figure 4.

Five first images 50 imaged while changing the light-emitting region on the display 120 are output to the standardizing unit 230 and thus standardized into images independent of the position and attitude of the camera unit 141.

The four indicators 14A, 14B, 14C, 14D provided on the tray 10 shown in Figure 5 are captured in each of the first images 50. The standardizing unit 230 obtains coordinates of the four indicators 14A, 14B, 14C, 14D in each of the first images 50, and obtains a projective transformation parameter from a relationship between the coordinates of the four indicators 14A, 14B, 14C, 14D and the four indicators 14A, 14B, 14C, and 14D having a known positional relationship with each other. The standardizing unit 230 standardizes the shape of each of the first images 50 (medicine 20) through projective transformation of the first images 50 by the projective transformation parameter thus obtained. In addition, since actual dimensions of the four indicators 14A, 14B, 14C, and 14D are also known, the size of the medicine 20 can also be standardized.

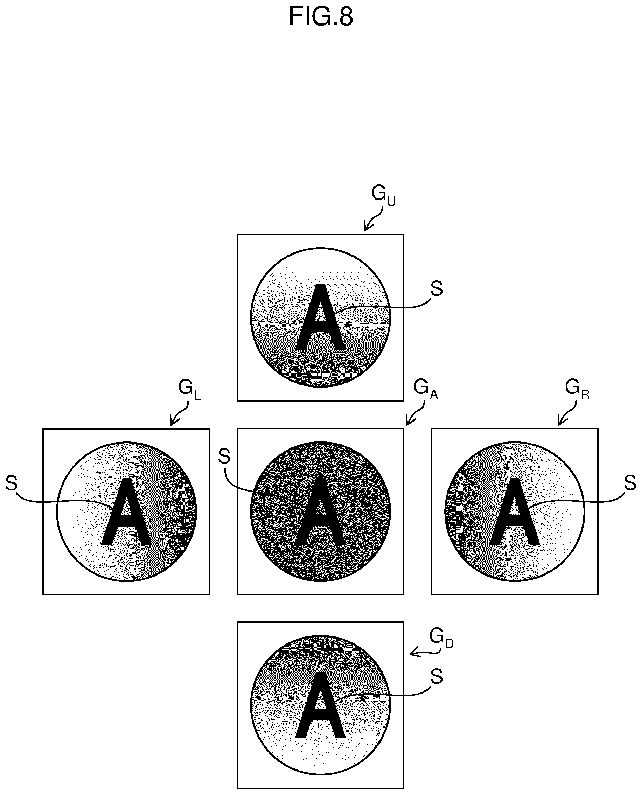

In addition, an example of the five first images after standardization is shown in Figure 8. Note that Figure 8 shows a part of the first images containing an image of the medicine 20, for the sake of simplification of explanation.

In Figure 8, four first images G_{L}, G_{R}, G_{U} and G_{D} are images taken in a state in which the left half, right half, upper half, and lower half of the display 120 are turned on, respectively with different illuminating directions of the illuminating light from the display 120, while first image G_{A} is an image taken in a state in which the display 120 is turned off.

The four first images G_{L}, G_{R}, Gu and G_{D} each have luminance unevenness generated along the illuminating direction. The symbol "A" on each of the images shown in Figure 8 indicates an embossed mark S. The embossed mark S on the images G_{L}, G_{R}, Gu and G_{D} is an indented pattern on the surface of the medicine, resulting in difference in the way in which a shadow of the embossed mark S appears depending on the illuminating direction of the illuminating light, as described later.

The standardizing unit 230 includes a generating unit that subtracts the first image G_{A} in a state in which the display 120 is turned off from each of the four first images G_{L}, G_{R}, Gu and G_{D} with different illumination directions of the display 120, to thereby generate four first images G_{L}, G_{R}, G_{U} and G_{D} with elimination of influence of ambient light. Since the four first images G_{L}, G_{R}, G_{U} and G_{D} with elimination of influence of ambient light by the generating unit are images illuminated only with the illuminating light from the display 120, the standardizing unit 230 adjusts white balance of the four first images G_{L}, G_{R}, G_{U} and G_{D} with a white balance gain corresponding to the color temperature (known color temperature) of the illuminating light of the display 120, to thereby make the color of the four first images G_{L}, G_{R}, G_{U} and G_{D} consistent to the original color of the medicine 20.

Note that, elimination or reduction of influence of ambient light may also be carried out by adjusting white balance, so that the known color of the tray 10 (for example, the color of the region in which the four indicators 14A, 14B, 14C, 14D are provided, preferably white) can be reproduced. In this case, it is not necessary to acquire the first image G_{A} in the state in which the display 120 is turned off.

The four first images G_{L}, G_{R}, Gu and G_{D}, in which the size and shape of the medicine 20 have been standardized and influence of ambient light has been eliminated or reduced by the standardizing unit 230, are output to the image extracting unit 240.

The image extracting unit 240 extracts (trims) a medicine region image from each of the four first images G_{L}, G_{R}, Gu and G_{D} thus standardized. The image extracting unit 240 is capable of trimming the medicine region image appropriately by utilizing the background color of the placement face 12 of the tray 10 on which the medicine 20 is placed as shown in Figure 5, or the background pattern of the placement face 12.

The four images G_{L}, G_{R}, G_{U} and G_{D} thus trimmed by the image extracting unit 240, different in the way in which a shadow of the embossed mark S appears, are each output to the image processing unit 270.

An edge image generating unit 274 of the image processing unit 270 generates four edge images from the four images G_{L}, G_{R}, G_{U} and G_{D}, by using edge extraction filters (for example, Sobel filters) of respective directions corresponding to the illuminating directions of the illuminating light.

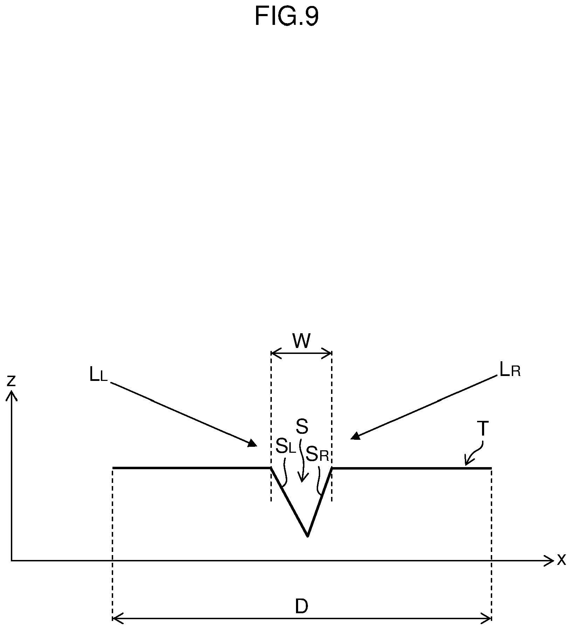

Figure 9 is a schematic view of a cross-sectional structure of a medicine T taken along an x-y plane passing through the center of the medicine T, showing a profile of a line of one pixel.

The medicine T shown in Figure 7 has a diameter D and provided with the embossed mark S, which is a score line composed of a groove having a V-shaped cross section, on the surface. The groove of the embossed mark S has a width W. Note that the width of the groove of the embossed mark S refers to a distance on the surface of the medicine T between one end and the other end of the groove in a direction orthogonal to the extending direction of the groove.

Here, the way in which a shadow of the embossed mark S appears is different between the case of using the left half of the display 120 as the light-emitting region and illuminating the medicine T with illuminating light L_{L} from the light-emitting region, and the case of using the right half of the display 120 as the light-emitting region and illuminating the medicine T with illuminating light L_{R} from the light-emitting region.

In other words, in the case of illuminating the medicine T with the illuminating light L_{L}, a right side face S_{R} of the embossed mark S is illuminated with the illuminating light L_{L} while a left side face S_{L} of the embossed mark S is not illuminated with the illuminating light L_{L}, generating a shadow on the left side face S_{L} of the embossed mark S. Similarly, in the case of illuminating the medicine T with the illuminating light L_{R} from the opposite direction of the illuminating light L_{L}, the left side face S_{L} of the embossed mark S is illuminated with the illuminating light L_{R} while the right side face S_{R} of the embossed mark S is not illuminated with the illuminating light L_{R}, generating a shadow on the right side face S_{R} of the embossed mark S.

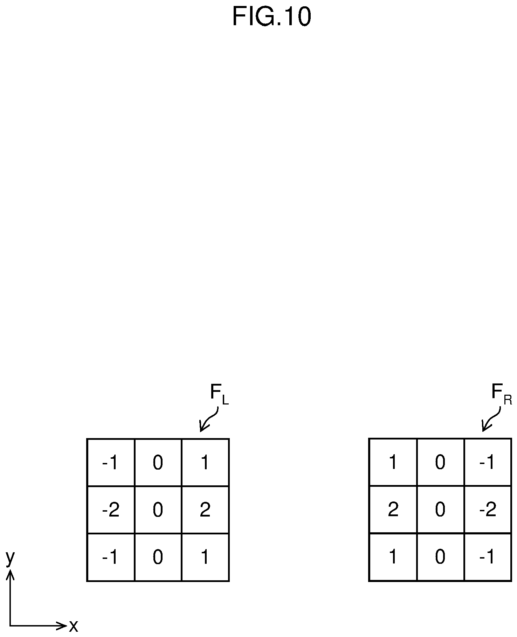

Figure 10 is a diagram showing an example of a Sobel Filter used for edge extraction by the edge image generating unit 274.

A Sobel filter F_{L} is used for edge extraction from the image G_{L} of the medicine T illuminated by the illuminating light L_{L} from the left side, while a Sobel filter F_{R} is used for edge extraction from the image G_{R} of the medicine T illuminated by the illuminating light L_{R} from the left side.

The kernel size of the Sobel filters F_{L} and F_{R} shown in Figure 10 is three pixels in x-axis direction × three pixels in y-axis direction in the present example, but is not limited thereto and it is preferable to change the kernel size depending on the resolution of the camera unit 141. In addition, as for the kernel size of the Sobel filters F_{L} and F_{R}, it is preferable to use a Sobel filter of a size greater than a half of (pixel count of) the width W of the embossed mark S. For example, when the pixel count of the width of the groove of the embossed mark S is four pixels, a Sobel filter of a size greater than two pixels, which is one-half thereof, is to be used. Using the edge extraction filter of a size selected in consideration of the pixel count of the width of the groove of the embossed mark S enables accurate extraction of the groove and, in addition, reduction of information other than the embossed mark, such as a pattern and scratches on the surface finer than the width of the groove. Note that a derivation method of a filter size and a filter coefficient of a Sobel filter is described in http://sssiii.seesaa.net/article/368842002.html.

The edge image generating unit 274 generates edge images corresponding to the images G_{L}, G_{R} by using the Sobel filters F_{L} and F_{R} for the images G_{L}, G_{R} respectively. The edge image generating unit 274 also generates edge images for the image Gu of the medicine T illuminated by the illuminating light from the upper side by using the upper half of the display 120 as the light-emitting region, and for the image G_{D} of the medicine T illuminated by the illuminating light from the lower side by using the lower half of the display 120 as the light-emitting region, by using the Sobel filters corresponding to the directions of the illuminating light similarly to the foregoing.

Note that the filter used for the edge extraction filter processing in an edge image synthesizing unit 272 is not limited to the Sobel filter, and the Laplacian filter, the Canny filter, and the like may also be used.

The edge image generating unit 274 outputs the four edge images generated respectively for the four images G_{L}, G_{R}, G_{U} and G_{D} to the edge image synthesizing unit 272 in the image processing unit 270.

Other inputs to the edge image synthesizing unit 272 include the four images G_{L}, G_{R}, G_{U} and G_{D} from the image extracting unit 240. The edge image synthesizing unit 272 generates an image with small luminance unevenness by, for example: selecting one image among the four images G_{L}, G_{R}, G_{U} and G_{D}; detecting luminance unevenness of the one image thus selected; and carrying out luminance unevenness correction process of, based on the luminance unevenness thus detected, correcting the luminance unevenness of the image selected. And then the edge image synthesizing unit 272 combines the four edge images generated by the edge image synthesizing unit 272 with the image with small luminance unevenness.

The image processing unit 270 can thus generate an image (second image) 60 having undergone an emphasis process for emphasizing the embossed mark in the image of the medicine with small luminance unevenness due to the illuminating light.

### <First Embodiment of Medicine Identifying Unit>

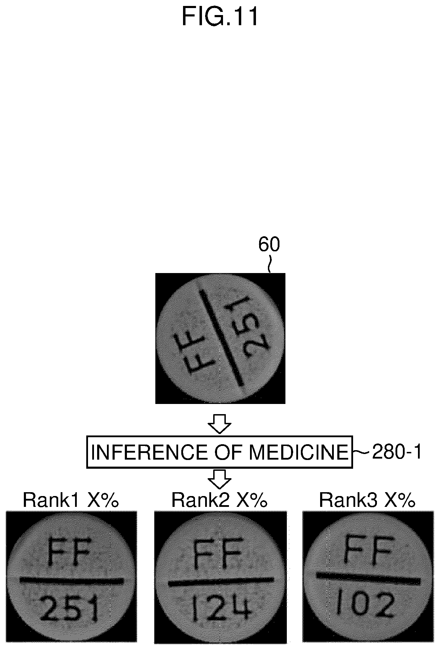

Figure 11 is a diagram showing an outline of a medicine identification process by a medicine identifying unit 280-1 according to the first embodiment of the medicine identifying unit 280 shown in Figure 4.

In Figure 11, 60 is a picture showing an example of the second image of the medicine with an emphasis on the embossed mark by the image processing unit 270.

The medicine identifying unit 280-1 infers which one of the registered medicines resembles the medicine to be identified, based on the second image 60. Deep learning (classification) is used for the inference. In the present example, the inference is carried out by medicine identifying unit 280-1, as described later in detail.

A result of the inference is output in a visually recognizable manner by an output unit such as a display device, a printer, and the like.

As the result of the inference, information (medicine identification information, medicine image, and the like) of the registered medicines can be output in an order of resemblance to the medicine to be identified (Rank 1, Rank 2, Rank 3, ...). Alternatively, information of the most resemblant medicine, or information of a plurality of medicines ranked in the predetermined top positions can be output.

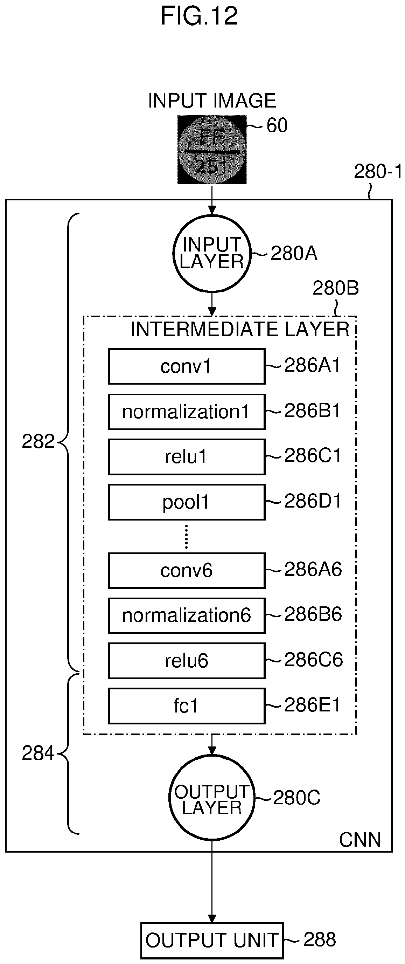

Figure 12 is a functional block diagram showing main functions of the medicine identifying unit 280-1 according to the first embodiment.

The medicine identifying unit 280-1 shown in Figure 12 is constituted of, in the present example, a convolutional neural network (CNN) 280-1, which is a mode of a machine learning device, and checks which one of the medicines having been registered (registered medicines) resembles the medicine to be identified. The CNN 280-1 is capable of preliminary learning by means of teacher data corresponding to each of the registered medicines, or additional learning by means of teacher data corresponding to a registered medicine to add.

It is preferable that the teacher data corresponding to the registered medicine is, for example, correct answer data containing a medicine image having undergone the emphasis process for emphasizing an embossed mark or a printed mark in an image taken of the registered medicine, the name of the registered medicine, and the like. Note that it is preferable that the teacher data having been used for, or to be used for, the learning is stored in the medicine DB 250 (Figure 4). The learning method by means of the teacher data is well-known, and therefore detailed description thereof is omitted herein.

The CNN 280-1 shown in Figure 12 functions as: a feature quantity calculating unit 282 configured to, when the second image 60 to be identified is used as an input image, extract (calculate) a feature quantity of the second image 60; and an inferring unit 284 configured to infer which one of the registered medicines resembles the medicine corresponding to the input image, based on the feature quantity thus calculated. The CNN 280-1 has a plurality of layered structures and retains a plurality of weighting parameters. The weighting parameter is exemplified by a filter coefficient of a filter called kernel used for convolution calculation in a convolutional layer, and the like.

The CNN 280-1 can be evolved from an unlearned model to a learned model that learns each registered medicine, through updating the weighting parameter from an initial value to an optimal value.

The CNN 280-1 is provided with: an input layer 280A; an intermediate layer 280B including a plurality of sets (in the present example, six sets) each configured with a convolutional layer 286A1, a normalization layer 286B1, an activation processing unit 286C1 using an activation function, and a pooling layer 286D1, and a fully connected layer 286E1; and an output layer 280C, in which each layer has a structure in which a plurality of "nodes" are connected by "edges".

The structure of the CNN 280-1 is not limited to that illustrated in Figure 12, and typical learning models such as VGG16, AlexNet, and the like may be employed.

The second image 60 of the medicine to be identified is input as the input image to the input layer 280A of the CNN 280-1. In the present example, the second image 60 is a colored image in RGB (Red Green Blue) color space, in other words three images of R, G, and B; therefore, the input image is an image set of three images in total.

The convolutional layers 286A1 to 286A6 play a role of feature extraction such as edge extraction from the image. The convolutional layers 285A1 to 286A6 play a role of feature extraction such as edge extraction from the image, through carrying out filter processing (carrying out convolution calculation using a filter) on the image set being input from the input layer 280A or on a proximate node in the previous layer and thus obtaining a "feature map".

The first convolutional layer 286A1 carries out the convolution calculation of the image set and a filter. Herein, since the image set has three channels of R image, G image, and B image (three images), for example in the case of a size-5 filter, the filter has a filter size of 5×5×3.

The convolution calculation using the 5×5×3 filter generates a one-channeled (for one image) "feature map" for each filter. Therefore, using N filters can generate a N-channeled "feature map".

A filter used in the second convolutional layer 285A2 (not illustrated) has, for example in the case of a size-3 filter, a filter size of 3×3×N.

The normalization layers 286B1 to 286B6 carry out normalization of luminance, contrast, etc. with respect not only to the input image, but also to the "feature map" in the middle of the CNN 280-1.

The activation processing units 286C1 to 286C6 processes an input signal by means of an activation function (for example, step function, sigmoid function, and softmax function), thus playing a role of preparing a value to pass on to the next layer.

The pooling layer 286D1 reduces the feature map being output from the convolutional layer 286A1 (in the present example, activation processing unit 286C1) to generate a new feature map. The "pooling layer" plays a role of imparting robustness to the feature extracted, not to be affected by parallel shift and the like.

One or more fully connected layer 286E1, which functions as the inferring unit 284, is weighted-connected to all nodes in the previous layer (in the present example, the feature map output from the activation processing units 286C6) and outputs a value (feature variable) converted by the activation function. A greater number of nodes leads to a greater number of divisions in a feature quantity space and a greater number of feature variables.

The output layer 280C, which functions as the inferring unit 284, carries out classification by converting the output (feature variable) from the fully connected layer 286E1 into a probability by using a softmax function, and maximizing the probability of correct classification into each region (in the present example, each medicine) (maximum likelihood estimation method). Note that the fully connected layer of the final phase may also be referred to as the output layer.

The output layer 280C outputs to an output unit 288 a result of inference indicating which one of the registered medicines resembles the second image 60 of the medicine (input image) as shown in Figure 11.

Information (medicine identification information, medicine image, and the like) of the registered medicines or the probabilities thereof can be output as the result of the inference from the output unit 288, in an order of resemblance to the medicine to be identified (Rank 1, Rank 2, Rank 3, ...).

A user can use the result of inference, which is output from the output unit 288, as assistance information for audit or discrimination of the medicine to be identified.

### <Second Embodiment of Medicine Identifying Unit>

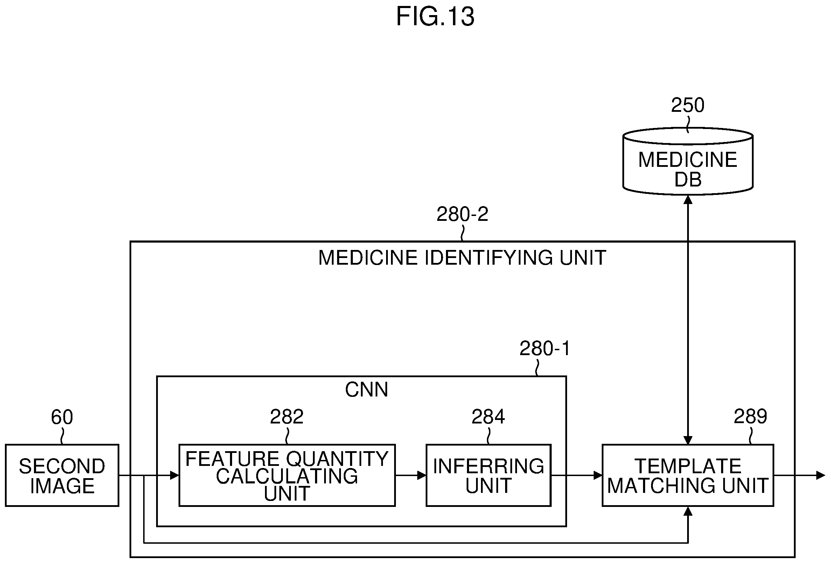

Figure 13 is a block diagram showing a medicine identifying unit 280-2 according to the second embodiment.

The medicine identifying unit 280-2 shown in Figure 13 is configured primarily with the CNN 280-1 and a template matching unit 289.

The CNN 280-1 is identical to that shown in Figure 12, and therefore detailed description thereof is omitted herein.

A result of inference by the CNN 280-1 is output to the template matching unit 289.

Other inputs to the template matching unit 289 include the second image 60 to be identified. The template matching unit 289 identifies the medicine through template matching between images of the registered medicines narrowed down from the registered medicines registered on the medicine DB 250 based on the result of inference from the CNN 280-1, and the second image 60. For example, images of medicines (images of medicines with an emphasis on embossed marks or printed marks) acquired in advance may be clustered. Then the CNN 280-1 infers a class in the clustering to which the second image 60 to be identified belongs, and the template matching unit 289 carries out template matching within the class thus inferred. The processing time of the template matching can thus be reduced.

### <Another Embodiment of Tray>

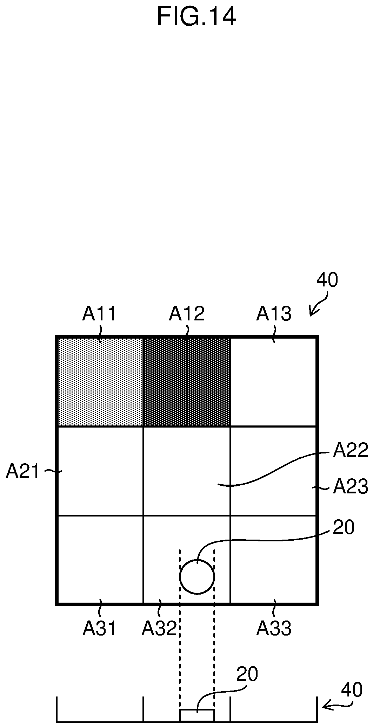

Figure 14 is a diagram showing another embodiment of the tray used in the medicine identification system according to the present invention.

A tray 40 shown in Figure 14 has a grid-shaped partition, and a medicine can be placed in each of nine regions A11, A12, A13, A21, A22, A23, A31, A32, A33 partitioned by the grid-shaped partition. Although the tray 40 is partitioned into the nine regions A11 to A33 by the grid-shaped partition, the number of regions to be partitioned by the partition is not limited thereto.

The size and shape of the grid-shaped partition on the tray 40 are known. In other words, the grid-shaped partition of which size and shape are known serves also as the indicator used for standardizing the size and shape of the medicine, similarly to the indicators 14A to 14D shown in Figure 5.

The color of the placement faces for the medicine in the nine regions A11 to A33 partitioned by the partition is different in at least one of hue, brightness, and saturation, for every placement face or for every plural placement faces.

Therefore, when a user places the medicine 20 to be identified in any one of the nine regions A11 to A33, it is preferable to place the medicine 20 on the placement face having the color different from the color of the medicine 20 to be identified.

For example, it is preferable that a medicine having a light color such as white is placed selectively on the placement face having a dark color such as black. This is for facilitating trimming of an image of a medicine region corresponding to the medicine 20 from the image taken by the camera unit 141.

Since the tray 40 has the placement faces in the nine regions A11 to A33, a plurality (up to nine) of medicines can be placed at the same time and the plurality of medicines can be imaged at the same time by the camera unit 141 of the smartphone 100.

In addition, in the case in which the medicine is provided with an embossed mark or a printed mark on both faces, it is preferable to, after imaging of one face of the medicine placed on the tray 40, turn over the medicine on the tray 40 and image the other face of the medicine. It is preferable to identify the medicine based on the second image generated from a plurality of images taken of the medicine before turning over and the second image generated from a plurality of images taken of the medicine after turning over. This enables use of information of the embossed mark or the printed mark provided on both faces of the medicine for identification of the medicine, leading to improvement in accuracy of identification of the medicine.

Note that the imaging method of the medicine by the smartphone 100 and the processing method of the image of the medicine thus imaged can be the same as in the case of imaging one medicine, therefore detailed description thereof is omitted herein.

### Medicine Identification Method

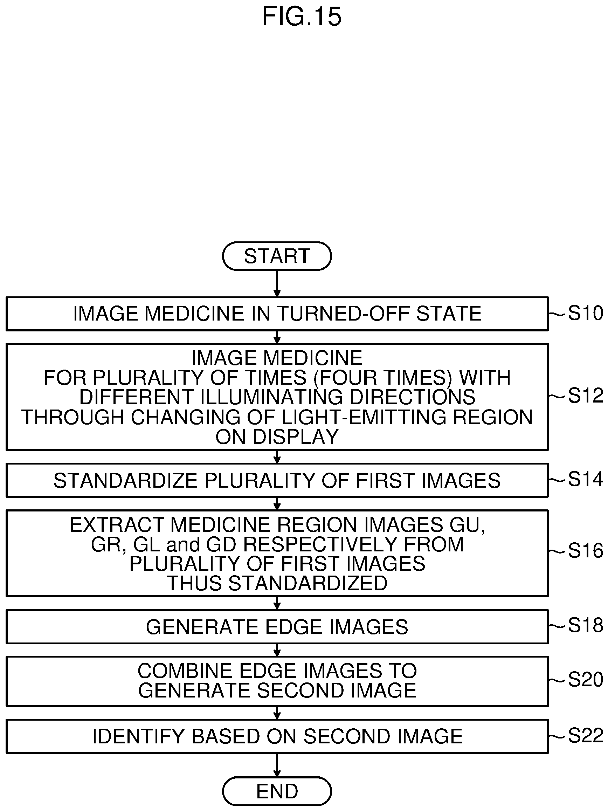

Figure 15 is a flowchart showing an embodiment of the medicine identification method according to the present invention, explaining processing procedure of each part of the medicine identification system shown in Figure 4.

In Figure 12, a user places the medicine 20 to be identified on the placement face 12 of the tray 10 (Figure 5). Subsequently, the user operates the smartphone 100 to cause the smartphone 100 to execute imaging of the medicine 20.

In other words, the display control unit 101A of the smartphone 100 puts the display 120 into a turned-off state, while the image acquiring unit 101B causes the camera unit 141 to execute imaging of the medicine 20 in a state in which the display 120 is turned off to acquire an image (first image) of the medicine 20 illuminated only with ambient light (Step S 10).

Subsequently, the display control unit 101A changes the light-emitting region on the display 120 sequentially (first step), and the image acquiring unit 101B causes the camera unit 141 to image for a plurality of times (in the present example, four times) the medicine 20 with different illuminating directions through changing of the light-emitting region on the display 120, to thereby acquire four images (first images) (second step S12).

The communication control unit 101C of the smartphone 100 transmits to the server 200 one first image taken in the state in which the display 120 is turned off acquired in Step S10, and four first images acquired in the second step S12 with different illuminating directions through changing of the light-emitting region on the display 120 (five first images 50 in total).

The standardizing unit 230 of the server 200 obtains coordinates of the four indicators 14A, 14B, 14C, 14D in the plurality of first images, and standardizes the shape and size of the plurality of first images (medicine 20) based on a relationship between the coordinates of the four indicators 14A, 14B, 14C, 14D and the four indicators 14A, 14B, 14C, and 14D having a known positional relationship with each other (third step S14). In addition, the standardizing unit 230 subtracts the first image G_{A} in a state in which the display 120 is turned off from each of the plurality of (four in the present example) first images G_{L}, G_{R}, Gu and G_{D} with different illumination directions of the display 120, to thereby generate a plurality of first images with elimination of influence of ambient light.

The image extracting unit 240 extracts (trims) first images G_{L}, G_{R}, G_{U} and G_{D} of a medicine region respectively from the plurality of (four in the present example) first images thus standardized (fourth step S16).

The edge image generating unit 274 of the image processing unit 270 generates edge images by using Sobel filters, which correspond respectively to the illuminating directions of the illuminating light, on the plurality of images G_{L}, G_{R}, Gu and G_{D} (Step S18).

The edge image synthesizing unit 272 generates an image with small luminance unevenness by: selecting one image among the plurality of images G_{L}, G_{R}, Gu and G_{D}; detecting luminance unevenness of the one image thus selected; and carrying out luminance unevenness correction process of, based on the luminance unevenness thus detected, correcting the luminance unevenness of the image selected. And then the edge image synthesizing unit 272 combines the plurality of edge images generated in Step S18 with the image with small luminance unevenness, to thereby generate a synthetic image (second image) (fifth step S20).

The medicine identifying unit 280 identifies the medicine 20 to be identified based on the second image thus generated (sixth step S22). The medicine identifying unit 280 outputs in a visually recognizable manner a result of the inference by the learned CNN 280-1 indicating which one of the registered medicines resembles the second image of the medicine (input image), and identifies the medicine through template matching by the template matching unit 289 between images of the registered medicines narrowed down by the result of inference and the second image and outputs an identification result in a visually recognizable manner.

### Others

In the present embodiment, four images of the medicine are taken with different illuminating directions through changing of the light-emitting region on the display; however, the number of images with different illuminating directions is not limited to four and only required to be three or more. In addition, the changing of the light-emitting region on the display is not limited to switching between the left half, right half, upper half, and lower half of the display, and it is only required that the changing of the light-emitting region on the display (including a high/low pattern of light emission intensity) results in different illumination directions to the medicine.

Furthermore, the medicine identification device is not limited to the server and may be any device that is communicable with the camera-equipped mobile terminal typified by a smartphone.

The hardware structure of the medicine identification device includes various processors described below. The various processors include: a CPU (Central Processing Unit) which is a general-purpose processor functioning as various control units through execution of software (program); a programmable logic device (PLD) such as an FPGA (Field Programmable Gate Array) which is a processor of which circuit configuration is changeable after production; a dedicated electrical circuit such as ASIC (Application Specific Integrated Circuit) which is a processor having a circuit configuration designed exclusively for execution of a specific processing; and the like.

One processing unit may be configured either with one of these various processors, or with a combination of two or more processors of the same type or different types (for example, a plurality of FPGAs, or a combination of a CPU and an FPGA). Alternatively, a plurality of control units may be configured with one processor. Configuration of a plurality of control units with one processor is exemplified by: a mode of configuring one processor with a combination of one or more CPU and software, as typified by a computer such as a client and a server, the processor functioning as a plurality of control units; and a mode of using a processor that realizes a function of an entire system including a plurality of control units by means of one IC (Integrated Circuit) chip, as typified by a System on Chip (SoC). As described above, the various control units are configured with one or more of the aforementioned various processors as a hardware structure.

In addition, in the present embodiment, the camera-equipped mobile terminal is provided with a function of taking a plurality of images with different illuminating directions; however, the camera-equipped mobile terminal may be provided with a part of the function of the medicine identification device (for example, functions of the standardizing unit, the image extracting unit, and the image processing unit), or an entirety of the function of the medicine identification device. In the latter case, the need for a medicine identification device separate from the camera-equipped mobile terminal is eliminated.

Furthermore, the medicine identification method by the medicine identifying unit is not limited to the present embodiment, and may be any method identifying a medicine based on an image (second image) with an emphasis on an embossed mark or a printed mark provided on the medicine.

The present invention further includes: a program that is installed on a general-purpose camera-equipped mobile terminal and realizes various functions (display control function, image acquiring function, and the like) as the camera-equipped mobile terminal according to the present invention; and a storage medium storing the program.

### Reference Signs List

2 Network
10, 40 Tray
12 Placement face
14A, 14B, 14C, 14D Indicators
20 Medicine
30 Ambient light
50 First image
60 Second image
100 Smartphone
101 Main control unit
101A Display control unit
101B Image acquiring unit
101C Communication control unit
102 Housing
110 Wireless communication unit
120 Display
121 Display panel
122 Operation panel
130 Phone-call unit
131 Speaker
132 Microphone
140 Operation unit
141 Camera unit
150 Storage unit
151 Internal storage unit
152 External storage unit
160 External input/output unit
170 GPS reception unit
180 Motion sensor unit
190 Power unit
200 Medicine identification device (Server)
210 Communication unit
220 CPU
230 Standardizing unit
240 Image extracting unit
250 Medicine DB
260 Memory
270 Image processing unit
272 Edge image synthesizing unit
274 Edge image generating unit
280 Medicine identifying unit
280-1 Medicine identifying unit (CNN)
280-2 Medicine identifying unit
280A Input layer
280B Intermediate layer
280C Output layer
282 Feature quantity calculating unit
284 Inferring unit
288 Output unit
289 Template matching unit
S Embossed mark

## Claims

1. A medicine identification system comprising:
a tray (10, 40) configured to have an indicator used for standardizing size and shape of a medicine, on which a medicine to be identified is to be placed;
a camera-equipped mobile terminal configured to include a camera on the same face as a display (120); and
a medicine identification device (200) configured to identify the medicine (20) based on an image taken by the camera-equipped mobile terminal,
wherein the camera-equipped mobile terminal is provided with:
a display control unit (101A) configured to, upon reception of an instruction input for medicine identification, change a light-emitting region on the display and change for a plurality of times an illuminating direction with respect to the medicine placed on the tray; and
an image acquiring unit (101B) configured to cause the camera to image for a plurality of times the medicine with different illuminating directions through changing of the light-emitting region on the display, to thereby acquire a plurality of first images of the medicine, and
the medicine identification device (200) is provided with:
a standardizing unit (230) configured to standardize each of the plurality of first images based on the indicator captured in the plurality of first images;
an image extracting unit (240) configured to extract a medicine region image from each of the plurality of first images thus standardized;
an image processing unit (270) configured to generate, based on a plurality of medicine region images thus extracted, a second image having undergone an emphasis process for emphasizing an embossed mark or a printed mark provided on the medicine; and
a medicine identifying unit (280) configured to identify the medicine based on the second image thus generated.

2. The medicine identification system according to claim 1, wherein the image processing unit (270) acquires a plurality of edge images from the plurality of medicine region images thus extracted by using edge extraction filters of respective directions corresponding to the illuminating directions, and generates the second image by using the plurality of edge images.

3. The medicine identification system according to claim 1 or 2, wherein:
the tray (10, 40) has a grid-shaped partition of which size and shape are known;
the medicine is placed on a placement face (12) partitioned by the grid-shaped partition; and
the grid-shaped partition serves also as the indicator, and
preferably,
color of the placement face (12) of the tray (10, 40) for the medicine partitioned by the grid-shaped partition is different in at least one of hue, brightness, and saturation, for every placement face or for every plural placement faces.

4. The medicine identification system according to any one of claims 1 to 3, wherein:
the image acquiring unit (101B) acquires, as the plurality of first images, three or more images with different illuminating directions and an image in a state in which the display is turned off; and
the medicine identification device (200) is provided with a generating unit configured to subtract the image in a state in which the display is turned off from each of the three or more images, to generate the plurality of first images with elimination of influence of ambient light.

5. The medicine identification system according to any one of claims 1 to 4, wherein:
the display (120) includes a polarizing plate; and
the camera includes a polarizing plate installed in front of an imaging lens, a polarization direction of the polarizing plate being different by 90° from a polarization direction of light emitted from the display (120).

6. The medicine identification system according to any one of claims 1 to 5, wherein the medicine identifying unit (280) includes: a feature quantity calculating unit (282) configured to calculate a feature quantity of the second image; and an inferring unit (284) configured to infer which one of registered medicines resembles the medicine based on the feature quantity of the second image, and
preferably,
the feature quantity (282) calculating unit and the inferring unit (284) comprise a learned convolutional neural network obtained through learning of each of the registered medicines by using as teacher data the second images corresponding to the registered medicines and medicine identification information for identifying the registered medicines, and
further preferably,
the medicine identifying unit (280) identifies the medicine through template matching between images of the registered medicines narrowed down by inference by the inferring unit and the second image.

7. The medicine identification system according to any one of claims 1 to 6, wherein the tray (10, 40) serves also as a protection cover for protecting the camera-equipped mobile terminal.

8. A medicine identification device for identifying a medicine to be identified based on an image taken by a camera-equipped mobile terminal,
wherein the camera-equipped mobile terminal is configured to:
include a camera on the same face as a display;
when imaging the medicine (20) to be identified placed on a tray (10, 40) having an indicator used for standardizing size and shape of a medicine, change a light-emitting region on the display;
change for a plurality of times an illuminating direction with respect to the medicine (20) placed on the tray; and
image by means of the camera for a plurality of times the medicine (20) with different illuminating directions through changing of the light-emitting region on the display, to acquire a plurality of first images of the medicine,
the medicine identification device (200) comprising:
an image accepting unit configured to accept the plurality of first images of the medicine from the camera-equipped mobile terminal;
a standardizing unit (230) configured to standardize each of the plurality of first images based on the indicator captured in the plurality of first images;
an image extracting unit (240) configured to extract a medicine region image from each of the plurality of first images thus standardized;
an image processing unit (270) configured to generate, based on a plurality of medicine region images thus extracted, a second image having undergone an emphasis process for emphasizing an embossed mark or a printed mark provided on the medicine; and
a medicine identifying unit (280) configured to identify the medicine based on the second image thus generated.

9. A medicine identification method for identifying a medicine (20) to be identified placed on a tray (10, 40) having an indicator used for standardizing size and shape of a medicine (20), comprising:
a first step in which a display control unit (101A) changes a light-emitting region on a display (120) of a camera-equipped mobile terminal including a camera on the same face as the display (120) , to thereby change for a plurality of times an illuminating direction with respect to the medicine placed on the tray;
a second step in which an image acquiring unit (101B) causes the camera to image for a plurality of times the medicine (20) with different illuminating directions through changing of the light-emitting region on the display (120), to thereby acquire a plurality of first images of the medicine;
a third step in which a standardizing unit (230) standardizes each of the plurality of first images based on the indicator captured in the plurality of first images;
a fourth step in which an image extracting unit (240) extracts a medicine region image from each of the plurality of first images thus standardized;
a fifth step in which an image processing unit (270) generates, based on a plurality of medicine region images thus extracted, a second image having undergone an emphasis process for emphasizing an embossed mark or a printed mark provided on the medicine; and
a sixth step in which a medicine identifying unit (280) identifies the medicine based on the second image thus generated.

10. The medicine identification method according to claim 9, wherein the fifth step acquires a plurality of edge images from the plurality of medicine region images thus extracted by using edge extraction filters of respective directions corresponding to the illuminating directions, and generates the second image by using the plurality of edge images.

11. The medicine identification method according to claim 9 or 10, wherein:
the tray (10, 40) has a grid-shaped partition of which size and shape are known;
the medicine (20) is placed on a placement face (12) partitioned by the grid-shaped partition; and
the grid-shaped partition serves also as the indicator, and
preferably,
color of the placement face (12) of the tray (10, 40) for the medicine (20) partitioned by the grid-shaped partition is different in at least one of hue, brightness, and saturation, for every placement face (12) or for every plural placement faces.

12. The medicine identification method according to any one of claims 9 to 11, wherein the second step acquires, as the plurality of first images, three or more images with different illuminating directions and an image in a state in which the display (120) is turned off, and
comprises a step in which a generating unit subtracts the image in a state in which the display is turned off from each of the three or more images, to thereby generate the plurality of first images with elimination of influence of ambient light.

13. The medicine identification method according to any one of claims 9 to 12, wherein: after the medicine (20) placed on the tray (10, 40) has been turned over, the first step to the fifth step carry out identical procedures respectively with respect to the medicine thus turned over; and
the sixth step identifies the medicine (20) based on the second images generated respectively for the medicine before turning over and the medicine after turning over.

14. A non-transitory and computer-readable storage medium in which a program installed on the camera-equipped mobile terminal according to any one of claims 1 to 7 is stored, wherein the program causes the camera-equipped mobile terminal to execute a medicine identification method,
wherein the medicine identification method includes:
a display control step of changing, upon reception of an instruction input for medicine identification, a light-emitting region on the display (120), and changing for a plurality of times an illuminating direction with respect to the medicine (20) placed on the tray (10, 40); and
an image acquiring step of causing the camera to image for a plurality of times the medicine (20) with different illuminating directions through changing of the light-emitting region on the display (120), to thereby acquire a plurality of first images of the medicine.

15. The non-transitory and computer-readable storage medium according to claim 14, wherein the medicine identification method further includes:
a standardizing step of standardizing each of the plurality of first images based on the indicator captured in the plurality of first images (50);
an image extracting step of extracting a medicine region image from each of the plurality of first images thus standardized;
an image processing step of generating, based on a plurality of medicine region images thus extracted, a second image (60) having undergone an emphasis process for emphasizing an embossed mark or a printed mark provided on the medicine (20); and
a medicine identifying step of identifying the medicine (20) based on the second image (60) thus generated.

## Patentansprüche

1. System zur Identifizierung von Medikamenten bzw. Arzneimitteln, das Folgendes umfasst:
ein Tablett (10, 40), das so konfiguriert ist, dass es einen Indikator aufweist, der zur Standardisierung von Größe und Form eines Medikaments verwendet wird, auf das ein zu identifizierendes Medikament gelegt werden soll;
ein mit einer Kamera ausgestattetes mobiles Endgerät, das so konfiguriert ist, dass es eine Kamera auf derselben Seite wie eine Anzeige (120) aufweist; und
eine Medikamenten-Identifikationsvorrichtung (200), die so konfiguriert ist, dass sie das Medikament (20) auf der Grundlage eines von dem mit einer Kamera ausgestatteten mobilen Endgerät aufgenommenen Bildes identifiziert,
wobei das mit einer Kamera ausgestattete mobile Endgerät aufweist:
eine Anzeigesteuereinheit (101A), die so konfiguriert ist, dass sie bei Empfang einer Befehlseingabe zur Medikamentenidentifikation einen lichtemittierenden Bereich auf der Anzeige ändert und mehrmals eine Beleuchtungsrichtung in Bezug auf das auf dem Tablett platzierte bzw. abgelegte Medikament ändert; und
eine Bilderfassungseinheit (101B), die so konfiguriert ist, dass sie die Kamera veranlasst, das Medikament mehrmals mit unterschiedlichen Beleuchtungsrichtungen durch Ändern des lichtemittierenden Bereichs auf der Anzeige abzubilden, um dadurch eine Vielzahl erster Bilder des Arzneimittels zu erfassen, und
wobei die Vorrichtung zur Identifizierung von Medikamenten (200) aufweist:
eine Standardisierungseinheit (230), die so konfiguriert ist, dass sie jedes der mehreren ersten Bilder auf der Grundlage des in den mehreren ersten Bildern erfassten Indikators standardisiert;
eine Bildextraktionseinheit (240), die so konfiguriert ist, dass sie aus jedem der mehreren ersten Bilder, die auf diese Weise standardisiert wurden, ein Bild des medizinischen Bereichs extrahiert;
eine Bildverarbeitungseinheit (270), die so konfiguriert ist, dass sie auf der Grundlage einer Vielzahl von auf diese Weise extrahierten Medikamentenbildern ein zweites Bild erzeugt, das einem Hervorhebungsprozess unterzogen wurde, um eine geprägte Markierung oder eine gedruckte Markierung hervorzuheben, die auf dem Medikament vorgesehen ist; und
eine Medikamentenidentifizierungseinheit (280), die so konfiguriert ist, dass sie das Medikament auf der Grundlage des so erzeugten zweiten Bildes identifiziert.

2. System zur Identifizierung von Medikamenten nach Anspruch 1, wobei die Bildverarbeitungseinheit (270) eine Vielzahl von Kantenbildern aus der Vielzahl der so extrahierten Bilder des Medikamentenbereichs unter Verwendung von Kantenextraktionsfiltern der jeweiligen Richtungen, die den Beleuchtungsrichtungen entsprechen, erfasst und das zweite Bild unter Verwendung der Vielzahl von Kantenbildern erzeugt.

3. System zur Identifizierung von Medikamenten nach Anspruch 1 oder 2, wobei:
das Tablett (10, 40) eine gitterförmige Trennwand, deren Größe und Form bekannt sind, aufweist;
das Medikament auf eine durch die gitterförmige Trennwand unterteilte Ablagefläche (12) gelegt wird; und
die gitterförmige Trennwand auch als Indikator dient, und
vorzugsweise,
die Farbe der Ablagefläche (12) des Tabletts (10, 40) für das durch die gitterförmige Trennwand unterteilte Medikament in mindestens einem der Parameter Farbton, Helligkeit und Sättigung für jede Ablagefläche oder für jede Mehrzahl von Ablageflächen unterschiedlich ist.

4. System zur Identifizierung von Medikamenten nach einem der Ansprüche 1 bis 3, wobei:
die Bilderfassungseinheit (101B) als die Vielzahl erster Bilder drei oder mehr Bilder mit unterschiedlichen Beleuchtungsrichtungen und ein Bild in einem Zustand erfasst, in dem die Anzeige ausgeschaltet ist; und
die Medikamenten-Identifizierungsvorrichtung (200) mit einer Erzeugungseinheit versehen ist, die so konfiguriert ist, dass sie das Bild in einem Zustand, in dem die Anzeige ausgeschaltet ist, von jedem der drei oder mehr Bilder subtrahiert, um die Vielzahl der ersten Bilder unter Eliminierung des Einflusses des Umgebungslichts zu erzeugen.

5. System zur Identifizierung von Medikamenten nach einem der Ansprüche 1 bis 4, wobei:
die Anzeige (120) eine polarisierende Platte enthält; und
die Kamera eine polarisierende Platte enthält, die vor einer Abbildungslinse installiert ist, wobei eine Polarisationsrichtung der polarisierenden Platte um 90° von einer Polarisationsrichtung des von der Anzeige (120) emittierten Lichts abweicht.

6. System zur Identifizierung von Medikamenten nach einem der Ansprüche 1 bis 5, wobei die Medikamenten-Identifikationseinheit (280) umfasst: eine MerkmalsmengenBerechnungseinheit (282), die so konfiguriert ist, dass sie eine Merkmalsmenge des zweiten Bildes berechnet; und eine Schlussfolgerungseinheit (284), die so konfiguriert ist, dass sie auf der Grundlage der Merkmalsmenge des zweiten Bildes schlussfolgert, welches der registrierten Medikamente dem Medikament ähnelt, und
vorzugsweise,
die Merkmalsmengenberechnungseinheit (282) und die Schlussfolgerungseinheit (284) ein gelerntes bzw. lernendes neuronales Faltungsnetzwerk umfassen, das durch Lernen jedes der registrierten Medikamente bzw. Arzneimittel erhalten wird, indem als Lehrerdaten die zweiten Bilder, die den registrierten Medikamenten entsprechen, und Medikamentenidentifikationsinformationen zum Identifizieren der registrierten Medikamente verwendet werden, und
weiter bevorzugt,
die Medikamenten-Identifizierungseinheit (280) identifiziert das Medikament durch Schablonenabgleich zwischen Bildern der registrierten Medikamente, die durch Ableitung durch die Ableitungseinheit eingegrenzt wurden, und dem zweiten Bild.

7. System zur Identifizierung von Medikamenten nach einem der Ansprüche 1 bis 6, wobei das Tablett (10, 40) auch als Schutzhülle zum Schutz des mit einer Kamera ausgestatteten mobilen Endgeräts dient.

8. Medikamenten-Identifizierungsvorrichtung zur Identifizierung eines zu identifizierenden Medikaments auf der Grundlage eines von einem mit einer Kamera ausgestatteten mobilen Endgerät aufgenommenen Bildes,
wobei das mit einer Kamera ausgestattete mobile Endgerät so konfiguriert ist, dass es:
eine Kamera auf der gleichen Seite bzw. Fläche wie eine Anzeige bzw. ein Display aufweist;
beim Abbilden des zu identifizierenden Medikaments (20), das auf einem Tablett (10, 40) mit einem Indikator zur Standardisierung von Größe und Form eines Medikaments platziert bzw. abgelegt ist, einen lichtemittierenden Bereich auf der Anzeige ändert;
die Beleuchtungsrichtung in Bezug auf das auf dem Tablett befindliche Arzneimittel (20) mehmals ändert; und
das Medikament (20) mittels der Kamera mehrmals bzw. für eine Vielzahl von Malen mit verschiedenen Beleuchtungsrichtungen durch Ändern des lichtemittierenden Bereichs auf der Anzeige abbildet, um eine Vielzahl von ersten Bildern des Medikaments zu erfassen,
wobei die Vorrichtung zur Identifizierung von Medikamenten (200) umfasst:
eine Bildannahmeeinheit, die so konfiguriert ist, dass sie die mehreren ersten Bilder des Medikaments von dem mit einer Kamera ausgestatteten mobilen Endgerät annimmt;
eine Standardisierungseinheit (230), die so konfiguriert ist, dass sie jedes der mehreren ersten Bilder auf der Grundlage des in den mehreren ersten Bildern erfassten Indikators standardisiert;
eine Bildextraktionseinheit (240), die so konfiguriert ist, dass sie aus jedem der mehreren ersten Bilder, die auf diese Weise standardisiert wurden, ein Bild des medizinischen Bereichs extrahiert;
eine Bildverarbeitungseinheit (270), die so konfiguriert ist, dass sie auf der Grundlage einer Vielzahl von auf diese Weise extrahierten Medikamentenbildern ein zweites Bild erzeugt, das einem Hervorhebungsprozess unterzogen wurde, um eine geprägte Markierung oder eine gedruckte Markierung, die auf dem Medikament bzw. Arzneimittel vorgesehen ist, hervorzuheben; und
eine Medikamentenidentifizierungseinheit (280), die so konfiguriert ist, dass sie das Medikament auf der Grundlage des so erzeugten zweiten Bildes identifiziert.

9. Verfahren zur Identifizierung von Medikamenten bzw. Arzneimitteln zum Identifizieren eines zu identifizierenden Medikaments (20), das auf einem Tablett (10, 40) mit einem Indikator zur Standardisierung von Größe und Form eines Medikaments (20) angeordnet ist, umfassend:
einen ersten Schritt, bei dem eine Anzeigesteuereinheit (101A) einen lichtemittierenden Bereich auf einer Anzeige (120) eines mit einer Kamera ausgestatteten mobilen Endgeräts, das eine Kamera auf derselben Seite bzw. Fläche wie die Anzeige (120) enthält, ändert, um dadurch mehrmals eine Beleuchtungsrichtung in Bezug auf das auf dem Tablett platzierte Medikament zu ändern;
einen zweiten Schritt, in dem eine Bilderfassungseinheit (101B) die Kamera veranlasst, das Medikament (20) mehrmals mit unterschiedlichen Beleuchtungsrichtungen durch Ändern des lichtemittierenden Bereichs auf der Anzeige (120) abzubilden, um dadurch eine Vielzahl erster Bilder des Medikaments zu erfassen;
einen dritten Schritt, in dem eine Standardisierungseinheit (230) jedes der mehreren ersten Bilder auf der Grundlage des in den mehreren ersten Bildern erfassten Indikators standardisiert;
einen vierten Schritt, in dem eine Bildextraktionseinheit (240) ein Bild einer medizinischen Region aus jedem der mehreren so standardisierten ersten Bilder extrahiert;
einen fünften Schritt, in dem eine Bildverarbeitungseinheit (270) auf der Grundlage einer Vielzahl von auf diese Weise extrahierten Medikamentenbildern ein zweites Bild erzeugt, das einem Hervorhebungsprozess unterzogen wurde, um eine geprägte Markierung oder eine gedruckte Markierung hervorzuheben, die auf dem Medikament vorgesehen ist; und
einen sechsten Schritt, in dem eine Medikamentenidentifizierungseinheit (280) das Medikament auf der Grundlage des so erzeugten zweiten Bildes identifiziert.

10. Verfahren zur Identifizierung von Medikamenten nach Anspruch 9, wobei der fünfte Schritt eine Vielzahl von Kantenbildern aus der Vielzahl der so extrahierten Bilder des Medikamentenbereichs unter Verwendung von Kantenextraktionsfiltern der jeweiligen Richtungen, die den Beleuchtungsrichtungen entsprechen, erfasst und das zweite Bild unter Verwendung der Vielzahl von Kantenbildern erzeugt.

11. Verfahren zur Identifizierung von Medikamenten nach Anspruch 9 oder 10, wobei:
sas Tablett (10, 40) eine gitterförmige Trennwand, deren Größe und Form bekannt sind aufweist;
das Medikament (20) auf einer durch die gitterförmige Trennwand unterteilten Ablagefläche (12) platziert wird; und
die gitterförmige Trennwand auch als Indikator dient, und
vorzugsweise,
die Farbe der Ablagefläche bzw. Bestückungsfläche (12) des Tabletts (10, 40) für das durch die gitterförmige Trennwand unterteilte Medikament (20) für jede Ablagefläche (12) oder für jede Mehrzahl von Ablageflächen in mindestens einem der Parameter Farbton, Helligkeit und Sättigung unterschiedlich ist.

12. Verfahren zur Identifizierung von Medikamenten nach einem der Ansprüche 9 bis 11, wobei der zweite Schritt als die Mehrzahl der ersten Bilder drei oder mehr Bilder mit unterschiedlichen Beleuchtungsrichtungen und ein Bild in einem Zustand, in dem die Anzeige (120) ausgeschaltet ist, erfasst, und
einen Schritt umfasst, in dem eine Erzeugungseinheit das Bild in einem Zustand, in dem die Anzeige ausgeschaltet ist, von jedem der drei oder mehr Bilder subtrahiert, um dadurch die Vielzahl der ersten Bilder unter Eliminierung des Einflusses von Umgebungslicht zu erzeugen.

13. Verfahren zur Identifizierung von Medikamenten nach einem der Ansprüche 9 bis 12, bei dem: nach dem Umdrehen des auf dem Tablett (10, 40) befindlichen Medikaments bzw. Arzneimittels (20) der erste Schritt bis zum fünften Schritt jeweils identische Verfahren in Bezug auf das so umgedrehte Medikament durchführen; und
der sechste Schritt identifiziert das Medikament (20) auf der Grundlage der zweiten Bilder, die jeweils für das Medikament vor dem Umdrehen und das Medikament nach dem Umdrehen erzeugt wurden.

14. Nicht-transitorisches und computerlesbares Speichermedium, in dem ein Programm, das auf dem mit einer Kamera ausgestatteten mobilen Endgerät nach einem der Ansprüche 1 bis 7 installiert ist, gespeichert ist, wobei das Programm das mit einer Kamera ausgestattete mobile Endgerät veranlasst, ein Verfahren zur Identifizierung von Medikamenten bzw. Arzneimitteln auszuführen,
wobei das Verfahren zur Identifizierung von Medikamenten Folgendes umfasst:
einen Anzeigesteuerungsschritt des Änderns eines lichtemittierenden Bereichs auf der Anzeige (120) bei Empfang einer Befehlseingabe zur Medikamentenidentifikation und des mehrmaligen Änderns einer Beleuchtungsrichtung in Bezug auf das auf dem Tablett (10, 40) platzierte Medikament (20); und
einen Bilderfassungsschritt, bei dem die Kamera veranlasst wird, das Medikament (20) mehrmals mit unterschiedlichen Beleuchtungsrichtungen durch Ändern des lichtemittierenden Bereichs auf der Anzeige (120) abzubilden, um dadurch eine Vielzahl erster Bilder des Medikaments zu erfassen.

15. Nichttransitorisches und computerlesbares Speichermedium nach Anspruch 14, wobei das Verfahren zur Identifizierung von Medikamenten ferner umfasst:
einen Standardisierungsschritt zum Standardisieren jedes der mehreren ersten Bilder auf der Grundlage des in den mehreren ersten Bildern (50) erfassten Indikators;
einen Bildextraktionsschritt des Extrahierens eines Bildes einer medizinischen Region aus jedem der Vielzahl der so standardisierten ersten Bilder;
einen Bildverarbeitungsschritt des Erzeugens eines zweiten Bildes (60), das einem Hervorhebungsprozess unterzogen wurde, um eine geprägte Markierung oder eine gedruckte Markierung, die auf dem Medikament (20) vorgesehen ist, hervorzuheben, auf der Grundlage einer Vielzahl von so extrahierten Bildern des Medikamentenbereichs; und
einen Schritt zur Identifizierung des Medikaments (20) auf der Grundlage des so erzeugten zweiten Bildes (60).

## Revendications

1. Système d'identification de médicaments comprenant :
un plateau (10, 40) configuré pour avoir un indicateur utilisé pour normaliser la taille et la forme d'un médicament, sur lequel un médicament à identifier doit être placé ;
un terminal mobile équipé d'une caméra configuré pour inclure une caméra sur la même face qu'un écran ou un affichage (120) ; et
un dispositif d'identification de médicament (200) configuré pour identifier le médicament (20) sur la base d'une image prise par le terminal mobile équipé d'une caméra,
dans lequel le terminal mobile équipé d'une caméra est pourvu de :
une unité de commande d'affichage (101A) configurée pour, à la réception d'une entrée d'instruction pour l'identification de médicaments, changer une région d'émission de lumière sur l'affichage et changer une pluralité de fois une direction d'éclairage par rapport au médicament placé sur le plateau ; et
une unité d'acquisition d'images (101B) configurée pour faire en sorte que l'appareil de prise de vues prenne plusieurs fois l'image du médicament avec différentes directions d'éclairage en changeant la région d'émission de lumière sur l'affichage, pour acquérir ainsi une pluralité de premières images du médicament, et
le dispositif d'identification de médicaments (200) est pourvu de :
une unité de normalisation (230) configurée pour normaliser chacune de la pluralité de premières images sur la base de l'indicateur capturé dans la pluralité de premières images ;
une unité d'extraction d'image (240) configurée pour extraire une image de région médicale de chacune de la pluralité de premières images ainsi normalisées ;
une unité de traitement d'image (270) configurée pour générer, sur la base d'une pluralité d'images de région de médicament ainsi extraites, une seconde image ayant subi un processus d'accentuation pour accentuer une marque en relief ou une marque imprimée prévue sur le médicament ; et
une unité d'identification de médicament (280) configurée pour identifier le médicament sur la base de la seconde image ainsi générée.

2. Système d'identification de médicament selon la revendication 1, dans lequel l'unité de traitement d'image (270) acquiert une pluralité d'images de bord à partir de la pluralité d'images de région de médicament ainsi extraites en utilisant des filtres d'extraction de bord de directions respectives correspondant aux directions d'éclairage, et génère la seconde image en utilisant la pluralité d'images de bord.

3. Système d'identification de médicaments selon la revendication 1 ou 2, dans lequel :
le plateau (10, 40) présente une cloison en forme de grille dont la taille et la forme sont connues ;
le médicament est placé sur une face de placement (12) cloisonnée par la cloison en forme de grille ; et
la cloison en forme de grille sert également d'indicateur, et
de préférence,
la couleur de la face de placement (12) du plateau (10, 40) pour le médicament cloisonné par la cloison en forme de grille est différente dans au moins l'une des teintes, luminosité et saturation, pour chaque face de placement ou pour chaque pluralité de faces de placement.

4. Système d'identification de médicaments selon l'une quelconque des revendications 1 à 3, dans lequel :
l'unité d'acquisition d'images (101B) acquiert, en tant que pluralité de premières images, trois images ou plus avec des directions d'éclairage différentes et une image dans un état dans lequel l'affichage est éteint ; et
le dispositif d'identification de médicaments (200) est muni d'une unité de génération configurée pour soustraire l'image dans un état dans lequel l'affichage est éteint de chacune des trois images ou plus, pour générer la pluralité de premières images avec élimination de l'influence de la lumière ambiante.

5. Système d'identification de médicaments selon l'une quelconque des revendications 1 à 4, dans lequel :
l'affichage (120) comprend une plaque de polarisation ; et
la caméra comprend une plaque de polarisation installée devant une lentille d'imagerie, une direction de polarisation de la plaque de polarisation étant différente de 90° d'une direction de polarisation de la lumière émise par l'affichage (120).

6. Système d'identification de médicaments selon l'une quelconque des revendications 1 à 5, dans lequel l'unité d'identification de médicaments (280) comprend : une unité de calcul de quantité de caractéristiques (282) configurée pour calculer une quantité de caractéristiques de la deuxième image ; et une unité d'inférence (284) configurée pour inférer lequel des médicaments enregistrés ressemble au médicament sur la base de la quantité de caractéristiques de la deuxième image, et
de préférence,
l'unité de calcul de quantité de caractéristiques (282) et l'unité d'inférence (284) comprennent un réseau neuronal convolutif appris obtenu par apprentissage de chacun des médicaments enregistrés en utilisant comme données d'apprentissage les secondes images correspondant aux médicaments enregistrés et les informations d'identification de médicaments pour identifier les médicaments enregistrés, et
de préférence encore,
l'unité d'identification de médicament (280) identifie le médicament par le biais d'une correspondance de modèle entre les images des médicaments enregistrés réduits par inférence par l'unité d'inférence et la seconde image.

7. Système d'identification de médicaments selon l'une quelconque des revendications 1 à 6, dans lequel le plateau (10, 40) sert également de couvercle de protection pour protéger le terminal mobile équipé d'une caméra.

8. Dispositif d'identification de médicament pour identifier un médicament à identifier sur la base d'une image prise par un terminal mobile équipé d'une caméra,
dans lequel le terminal mobile équipé d'une caméra est configuré pour :
inclure une caméra sur la même face que l'écran ;
lors de l'imagerie du médicament (20) à identifier placé sur un plateau (10, 40) ayant un indicateur utilisé pour normaliser la taille et la forme d'un médicament, changer une région d'émission de lumière sur l'affichage ;
changer plusieurs fois une direction d'éclairage par rapport au médicament (20) placé sur le plateau ; et
imager au moyen de la caméra une pluralité de fois le médicament (20) avec différentes directions d'éclairage en changeant la région d'émission de lumière sur l'affichage, pour acquérir une pluralité de premières images du médicament,
le dispositif d'identification de médicaments (200) comprenant :
une unité d'acceptation d'image configurée pour accepter la pluralité de premières images du médicament à partir du terminal mobile équipé d'une caméra ;
une unité de normalisation (230) configurée pour normaliser chacune de la pluralité de premières images sur la base de l'indicateur capturé dans la pluralité de premières images ;
une unité d'extraction d'image (240) configurée pour extraire une image de région médicale de chacune de la pluralité de premières images ainsi normalisées ;
une unité de traitement d'image (270) configurée pour générer, sur la base d'une pluralité d'images de région de médicament ainsi extraites, une seconde image ayant subi un processus d'accentuation pour accentuer une marque en relief ou une marque imprimée prévue sur le médicament ; et
une unité d'identification de médicament (280) configurée pour identifier le médicament sur la base de la seconde image ainsi générée.

9. Procédé d'identification de médicament pour identifier un médicament (20) à identifier placé sur un plateau (10, 40) ayant un indicateur utilisé pour normaliser la taille et la forme d'un médicament (20), comprenant :
une première étape dans laquelle une unité de commande d'affichage (101A) change une région d'émission de lumière sur un affichage (120) d'un terminal mobile équipé d'une caméra comprenant une caméra sur la même face que l'affichage (120), pour changer ainsi une pluralité de fois une direction d'éclairage par rapport au médicament placé sur le plateau ;
une deuxième étape dans laquelle une unité d'acquisition d'images (101B) fait en sorte que l'appareil de prise de vues prenne une image plusieurs fois du médicament (20) avec différentes directions d'éclairage en changeant la région d'émission de lumière sur l'affichage (120), pour acquérir ainsi une pluralité de premières images du médicament ;
une troisième étape dans laquelle une unité de normalisation (230) normalise chacune de la pluralité de premières images sur la base de l'indicateur capturé dans la pluralité de premières images ;
une quatrième étape dans laquelle une unité d'extraction d'image (240) extrait une image de région médicale de chacune de la pluralité de premières images ainsi normalisées ;
une cinquième étape dans laquelle une unité de traitement d'image (270) génère, sur la base d'une pluralité d'images de région de médicament ainsi extraites, une seconde image ayant subi un processus d'accentuation pour accentuer une marque en relief ou une marque imprimée prévue sur le médicament ; et
une sixième étape dans laquelle une unité d'identification de médicament (280) identifie le médicament sur la base de la seconde image ainsi générée.

10. Procédé d'identification de médicament selon la revendication 9, dans lequel la cinquième étape acquiert une pluralité d'images de bord à partir de la pluralité d'images de région de médicament ainsi extraites en utilisant des filtres d'extraction de bord de directions respectives correspondant aux directions d'éclairage, et génère la seconde image en utilisant la pluralité d'images de bord.

11. Procédé d'identification de médicaments selon la revendication 9 ou 10, dans laquelle :
le plateau (10, 40) présente une cloison en forme de grille dont la taille et la forme sont connues ;
le médicament (20) est placé sur une face de placement (12) cloisonnée par la cloison en forme de grille ; et
la cloison en forme de grille sert également d'indicateur, et
de préférence,
couleur de la face de placement (12) du plateau (10, 40) pour le médicament (20) divisé par la séparation en forme de grille est différente dans au moins l'une des teintes, luminosité et saturation, pour chaque face de placement (12) ou pour chaque pluralité de faces de placement.

12. Procédé d'identification de médicaments selon l'une quelconque des revendications 9 à 11, dans lequel la deuxième étape acquiert, en tant que pluralité de premières images, trois images ou plus avec des directions d'éclairage différentes et une image dans un état dans lequel l'affichage (120) est éteint, et
comprend une étape dans laquelle une unité de génération soustrait l'image dans un état dans lequel l'affichage est éteint de chacune des trois images ou plus, pour générer ainsi la pluralité de premières images avec élimination de l'influence de la lumière ambiante.

13. Procédé d'identification de médicaments selon l'une quelconque des revendications 9 à 12, dans lequel : après que le médicament (20) placé sur le plateau (10, 40) a été retourné, la première étape à la cinquième étape effectuent des procédures identiques respectivement par rapport au médicament ainsi retourné ; et
la sixième étape identifie le médicament (20) sur la base des secondes images générées respectivement pour le médicament avant le retournement et le médicament après le retournement.

14. Support de stockage non-transitoire et lisible par ordinateur dans lequel un programme installé sur le terminal mobile équipé d'une caméra selon l'une quelconque des revendications 1 à 7 est stocké , dans lequel le programme amène le terminal mobile équipé d'une caméra à exécuter un procédé d'identification de médicament,
dans lequel la méthode d'identification des médicaments comprend :
une étape de commande d'affichage consistant à changer, à la réception d'une entrée d'instruction pour l'identification d'un médicament, une région d'émission de lumière sur l'affichage (120), et à changer une pluralité de fois une direction d'éclairage par rapport au médicament (20) placé sur le plateau (10, 40) ; et
une étape d'acquisition d'image consistant à amener la caméra à imager une pluralité de fois le médicament (20) avec différentes directions d'éclairage en changeant la région d'émission de lumière sur l'affichage (120), pour acquérir ainsi une pluralité de premières images du médicament.

15. Support de stockage non-transitoire et lisible par ordinateur selon la revendication 14, dans lequel le procédé d'identification de médicaments comprend en outre :
une étape de normalisation consistant à normaliser chacune de la pluralité de premières images sur la base de l'indicateur capturé dans la pluralité de premières images (50) ;
une étape d'extraction d'image consistant à extraire une image de région médicale de chacune de la pluralité de premières images ainsi normalisées ;
une étape de traitement d'image consistant à générer, sur la base d'une pluralité d'images de région de médicament ainsi extraites, une seconde image (60) ayant subi un processus d'accentuation pour accentuer une marque en relief ou une marque imprimée prévue sur le médicament (20) ; et
une étape d'identification de médicament consistant à identifier le médicament (20) sur la base de la seconde image (60) ainsi générée.
